(19) Europäisches Patentamt / European Patent Office / Office européen des brevets

(11) **EP 4 654 204 A1**

(12) **EUROPEAN PATENT APPLICATION**
published in accordance with Art. 153(4) EPC

(43) Date of publication:
26.11.2025 Bulletin 2025/48

(21) Application number: 24744411.0

(22) Date of filing: **19.01.2024**

(51) International Patent Classification (IPC):
**G16B 15/00** (2019.01)

(52) Cooperative Patent Classification (CPC):
**G16B 15/00**

(86) International application number:
**PCT/CN2024/073357**

(87) International publication number:
**WO 2024/153242 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 19.01.2023 CN 202310085702
24.07.2023 CN 202310908572

(71) Applicant: **Biomap (Beijing) Intelligence Technology Limited**
**Beijing 100089 (CN)**

(72) Inventors:
• LUO, Yujie
  **Beijing 100089 (CN)**
• SUN, Yiwu
  **Beijing 100089 (CN)**
• LI, Shaochuan
  **Beijing 100089 (CN)**
• LI, Hui
  **Beijing 100089 (CN)**
• SONG, Le
  **Beijing 100089 (CN)**

(74) Representative: **Elzaburu S.L.P.**
**Edificio Torre de Cristal**
**Paseo de la Castellana 259 C, planta 28**
**28046 Madrid (ES)**

(54) **TRAINING METHOD FOR PROTEIN COMPLEX STRUCTURE PREDICTION MODEL, DEVICE, AND MEDIUM**

(57) A training method for a protein complex structure prediction model, a protein complex structure prediction method, an electronic device, a computer-readable medium, and a program product. The training method comprises: acquiring a real distance map corresponding to current training data; acquiring input features corresponding to the current training data; inputting the input features into a protein complex structure prediction model comprising current parameters, to obtain a prediction result; and updating the current parameters according to a loss function, so as to obtain the protein complex structure prediction model by training, wherein the loss function comprises a distance loss function, the distance loss function is used for representing the mapping relationship between a first difference and parameters of the protein complex structure prediction model, and the first difference is the difference between a predicted distance map and the real distance map.

FIG. 1

## Description

### TECHNICAL FIELD

The present disclosure relates to the field of artificial intelligence, and particularly relates to a method for training a protein complex structure prediction model, a method for protein complex structure prediction, an electronic device, a computer-readable storage medium, and a computer program product.

### BACKGROUND

Recently, deep learning techniques have shown significant improvements in computational speed and accuracy in the field of protein complex structure prediction. For example, EquiDock is a pioneering method that applies deep learning to rigid protein docking, and AlphaFold Multimer is hailed as a breakthrough for directly predicting complex structures from amino acid sequences. However, when predicting protein complex structures, EquiDock relies solely on structural modality information and does not utilize sequence modality information. AlphaFold Multimer, while considering sequence modality information, fails to effectively leverage the characteristic that protein monomers to be complexed can be regarded as rigid bodies, leading to inaccurate prediction results in certain situations.

The methods described in this section are not necessarily methods that have been previously conceived or adopted. Unless otherwise indicated, it should not be assumed that any method described in this section is considered prior art merely because it is included in this section. Similarly, unless otherwise indicated, problems mentioned in this section should not be considered to have been recognized in any prior art.

### SUMMARY

According to the first aspect of the present disclosure, a method for training a protein complex structure prediction model is provided, comprising: obtaining a ground truth distance map corresponding to current training data; wherein the current training data comprises a first amino acid sequence of a first protein, a second amino acid sequence of a second protein, monomeric three-dimensional (3D) structural information of the first protein, monomeric 3D structural information of the second protein, and 3D structural information of a ground truth protein complex composed of the first protein and the second protein; wherein the monomeric 3D structural information of the first protein comprises coordinates of reference atoms included in each amino acid residue of the first protein; the monomeric 3D structural information of the second protein comprises coordinates of reference atoms included in each amino acid residue of the second protein; the 3D structural information of the ground truth protein complex comprises first ground truth coordinates of reference atoms included in each amino acid residue of the first protein and second ground truth coordinates of reference atoms included in each amino acid residue of the second protein in the ground truth protein complex; and the ground truth distance map is determined based on the first ground truth coordinates and the second ground truth coordinates; obtaining input features corresponding to the current training data; wherein the input features are determined based on the first amino acid sequence, the second amino acid sequence, the monomeric 3D structural information of the first protein, and the monomeric 3D structural information of the second protein; performing model prediction, comprising: inputting the input features into a protein complex structure prediction model comprising current parameters to obtain a prediction result; wherein the prediction result comprises a predicted distance map; the predicted distance map and the ground truth distance map comprise two-dimensional (2D) matrices, wherein an element at an i-th row and a j-th column of the predicted distance map represents a predicted distance between an i-th reference atom of the first protein and a j-th reference atom of the second protein when the first protein and the second protein form a protein complex, as predicted by the protein complex structure prediction model; and an element at the i-th row and the j-th column of the ground truth distance map represents a ground truth distance between the i-th reference atom of the first protein and the j-th reference atom of the second protein in the ground truth protein complex; updating the current parameters based on a loss function to train and obtain the protein complex structure prediction model; wherein the loss function comprises a distance loss function; the distance loss function is used to characterize a mapping relationship between a first difference and the parameters of the protein complex structure prediction model; and the first difference is a difference between the predicted distance map and the ground truth distance map.

According to the second aspect of the present disclosure, a method for predicting protein complex structure is provided, obtaining input data, wherein the input data comprises a first target amino acid sequence of a first target protein, a second target amino acid sequence of a second target protein, monomeric three-dimensional (3D) structural information of the first target protein, monomeric 3D structural information of the second target protein, wherein the monomeric 3D structural information of the first target protein comprises coordinates of reference atoms included in each amino acid residue of the first target protein; the monomeric 3D structural information of the second target protein comprises coordinates of reference atoms included in each amino acid residue of the second target protein; determining input features corresponding to the input data based on the first target amino acid sequence, the second target amino acid sequence, the monomeric 3D structural information of the first target protein, and the monomeric 3D structural information of the second target protein; inputting the input features corresponding to the input data into the protein complex structure prediction model trained by the method according to the first aspect of the present disclosure to obtain a prediction result; wherein the prediction result comprises a predicted distance map; the predicted distance map comprise 2D matrices, wherein an element at an i-th row and a j-th column of the predicted distance map represents a predicted distance between an i-th reference atom of the first target protein and a j-th reference atom of the second target protein when the first target protein and the second target protein form a protein complex, as predicted by the protein complex structure prediction model; determining an optimal docking pose of the first target protein and the second target protein, wherein when the first target protein and the second target protein are docked into a protein complex in the optimal docking pose, a difference between distances between reference atoms included in each amino acid residue of the first target protein and reference atoms included in each amino acid residue of the second target protein and a target predicted distance map is less than a difference when docked in a non-optimal docking pose, and the target predicted distance map is determined based on the predicted distance map; taking a 3D structure of the protein complex obtained by docking the first target protein and the second target protein in the optimal docking pose as a predicted 3D structure of the protein complex composed of the first target protein and the second target protein.

According to another aspect of the present disclosure, an electronic device is provided, comprising: a processor; and a memory, wherein the memory stores instructions executable by the processor, which, when executed by the processor, cause the processor to perform the method according to any one of the above aspects.

According to another aspect of the present disclosure, a non-transitory computer-readable storage medium storing instructions is provided, wherein the instructions, when executed by a processor, cause the processor to perform the method according to any one of the above aspects.

According to another aspect of the present disclosure, a computer program product is provided, comprising: instructions, wherein the instructions, when executed by a processor, cause the processor to perform the method according to any one of the above aspects.

These and other aspects of the present disclosure will be apparent from and elucidated with reference to the examples described hereinafter.

## BRIEF DESCRIPTION OF THE DRAWINGS

The accompanying drawings exemplarily illustrate examples and constitute a part of the specification, and are used in conjunction with the textual description of the specification to explain exemplary implementations of the examples. The illustrated examples are for illustrative purposes only and do not limit the scope of the claims. In all figures, identical reference numerals refer to similar but not necessarily identical elements.

FIG. 1 is a flowchart of a method for training a protein complex structure prediction model according to an example of the present disclosure;

FIG. 2 is a schematic diagram of rigid protein docking according to an example of the present disclosure;

FIG. 3 is a schematic diagram of a method for processing input features by a protein complex structure prediction model in a method for training a protein complex structure prediction model according to an example of the present disclosure;

FIG. 4A is a flowchart of a method for training a protein complex structure prediction model according to another example of the present disclosure;

FIG. 4B is a flowchart of a method for predicting a protein complex structure according to another example of the present disclosure;

FIG. 5 is a flowchart of a method for predicting a protein complex structure according to an example of the present disclosure;

FIG. 6 is a flowchart of a method for predicting a protein complex structure according to another example of the present disclosure;

FIG. 7 is a block diagram of an exemplary electronic device that can be used to implement an example of the present disclosure.

**DETAILED DESCRIPTION**

In the present disclosure, unless otherwise specified, the terms "first", "second", etc., are used to describe various elements and are not intended to limit the positional, temporal, or importance relationships of these elements. Such terms are merely used to distinguish one element from another. In some examples, a first element and a second element may refer to the same instance of an element, while in some cases, based on the context, they may refer to different instances.
The terminology used in the description of various examples in the present disclosure is merely for the purpose of describing specific examples and is not intended to be limiting. Unless the context clearly indicates otherwise, if the quantity of an element is not specifically limited, the element may be one or more. As used herein, the term "plurality" means two or more, and the term "based on" should be interpreted as "at least partially based on". Furthermore, the terms "and/or" and "at least one of..." cover any one of the listed items as well as all possible combinations.
Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning consistent with their meaning in the relevant art and/or in the context of the present specification, and will not be interpreted in an idealized or overly formal sense unless expressly so defined herein.

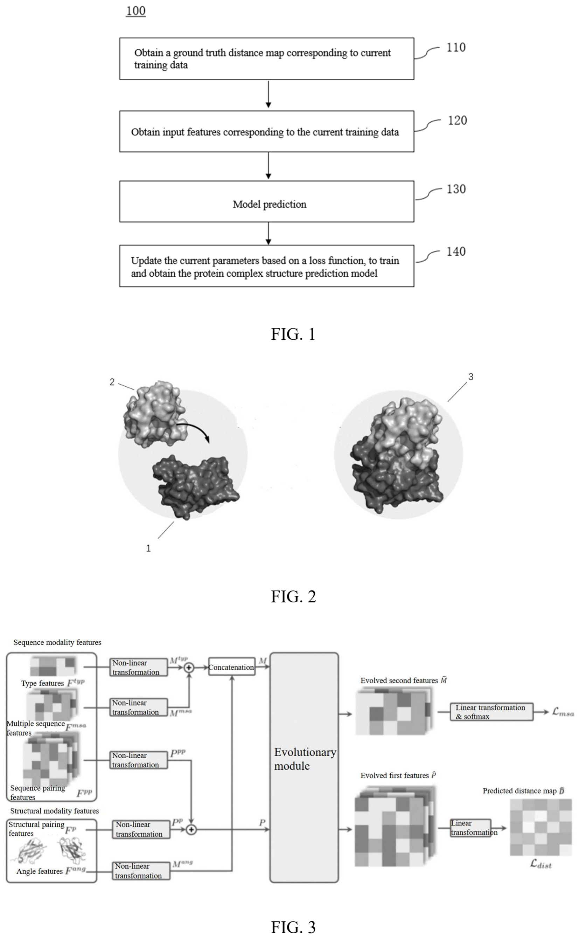

FIG. 1 is a flowchart of a method 100 for training a protein complex structure prediction model according to an example of the present disclosure. As shown in FIG. 1, the method 100 includes steps 110 to 140.
In step 110, a ground truth distance map corresponding to current training data is obtained.
The current training data may be one or more entries. If there are multiple entries of current training data, a ground truth distance map corresponding to each entry of current training data is obtained.
One entry of current training data comprises a first amino acid sequence of a first protein and a second amino acid sequence of a second protein, monomeric three-dimensional (3D) structural information of the first protein, monomeric 3D structural information of the second protein, and 3D structural information of a ground truth protein complex composed of the first protein and the second protein. In other words, the training data includes both amino acid sequences of proteins and 3D structural information of proteins, providing multi-modal information including sequence modality and structural modality to the model, which is conducive to the model to make accurate predictions.
The protein in the present disclosure should be understood in a broad sense, and polypeptides are also included in the definition of protein in the present disclosure. The protein in the present disclosure can be an antibody, such as a heavy chain or light chain of an antibody, or other proteins. A protein complex is a complex formed by two or more functionally related polypeptide chains through disulfide bonds or other protein interactions. A protein complex is composed of multiple protein monomers (protein monomers are also referred to as protein chains hereinafter). The protein complex in the present disclosure can be a complex of antibody heavy chain and light chain, a complex of an antibody and other proteins (other proteins refer to non-antibody proteins, such as antigens), or a complex of other proteins.
It can be understood that, in examples of the present disclosure, when predicting the 3D structure of a protein complex through protein monomer docking, the first protein and the second protein can be regarded as rigid bodies. The position and pose of one of the first protein and the second protein remain fixed, while the other adjusts its position and pose with a docking pose (the docking pose includes translation and rotation) to dock with the first protein to form a protein complex.
FIG. 2 is a schematic diagram showing the first protein 1 remaining fixed, and the second protein 2 adjusting its docking pose to dock with the first protein 1 to form a protein complex 3.
3D structural information is used to characterize the 3D structure of a protein or protein complex. Generally, monomeric 3D structural information usually comprises the coordinates of atoms included in a protein monomer, and 3D structural information of a protein complex comprises the coordinates of atoms included in a protein complex. To reduce computational complexity, the monomeric 3D structure and complex 3D structure in examples of the present disclosure may not include the coordinates of all atoms, but only the coordinates of reference atoms. Reference atoms can be at least some backbone atoms in amino acid residues, such as $C\beta$ atoms ($C\alpha$ atoms can be used instead for amino acids that do not contain $C\beta$ atoms), etc. For example, the monomeric 3D structural information of the first protein in the current training data comprises the coordinates of reference atoms included in each amino acid residue of the first protein; the monomeric 3D structural information of the second protein comprises the coordinates of reference atoms included in each amino acid residue of the second protein; the 3D structural information of the ground truth protein complex comprises first ground truth coordinates of reference atoms included in each amino acid residue of the first protein and second ground truth coordinates of reference atoms included in each amino acid residue of the second protein in the ground truth protein complex. Exemplarily, in addition to the coordinates of reference atoms, the 3D structural information may also comprise coordinates of other backbone atoms and side chain atoms not used as reference atoms.
Exemplarily, the monomeric 3D structural information and the 3D structural information of the ground truth protein complex can be processed from 3D structural information of protein complexes obtained from the Protein Data Bank (PDB) database. The 3D structural information of protein complexes in the PDB database can be determined by experimental means of X-ray crystallography and cryo-electron microscopy, etc. and it contains information of the atom number, coordinates, the type of amino acid residue where the atom is located, and the number of the protein chain where the atom is located, etc. The specific processing can be as follows: filter out the required information from the 3D structural information of protein complexes in the PDB database (e.g., filter out the coordinates of reference atoms included in the first protein and the coordinates of reference atoms included in the second protein of the protein complex), and then perform coordinate transformation on the filtered information to obtain the monomeric 3D structural information of the first protein, the monomeric 3D structural information of the second protein, and the 3D structural information of the ground truth protein complex. The purpose of coordinate transformation is to facilitate subsequent calculations. For example, if during subsequent protein docking, the first protein is fixed and the docking pose of the second protein is adjusted to dock with the first protein to form a protein complex, coordinate transformation can be performed to make the centroid of the first protein (the centroid can be the average of the coordinates of reference atoms included in each amino acid residue of the protein) in the filtered information located at the coordinate origin. The coordinates of the atoms included in the first protein in the filtered information after the coordinate transformation are used as the monomeric 3D structural information of the first protein, and the filtered information after the coordinate transformation is used as the 3D structural information of the ground truth protein complex. The coordinates of the atoms included in the second protein in the filtered information that have not undergone coordinate transformation can be used as the monomeric 3D structural information of the second protein, or the coordinates of the atoms included in the second protein in the filtered information that have not undergone coordinate transformation can be subjected to coordinate transformation to make the centroid of the second protein also located at the coordinate origin, and the coordinates of the atoms included in the second protein after coordinate transformation are used as the monomeric 3D structural information of the second protein.

Exemplarily, the ground truth distance map can be calculated from 3D structural information of protein complexes obtained from the PDB (Protein Data Bank) database, or it can be calculated based on the filtered information, or it can be calculated based on the first ground truth coordinates and the second ground truth coordinates in the 3D structural information of the ground truth protein complex. When the first protein and the second protein contain m and n amino acid residues respectively, the ground truth distance map can include an m*n 2D matrix. An element at the i-th row and the j-th column (i=1..m, j=1..n) of the ground truth distance map represents the ground truth distance between the i-th reference atom of the first protein and the j-th reference atom of the second protein in the ground truth protein complex. Exemplarily, when calculating the element values in the ground truth distance map, the first ground truth coordinates of the i-th reference atom of the first protein and the second ground truth coordinates of the j-th reference atom of the second protein can be found from the 3D structural information of the ground truth protein complex, and then the distance between the two ground truth coordinates is calculated. The element value of the element in the ground truth distance map is determined based on this distance, and the specific determination method will be described later. For example, if the coordinates of the first ground truth coordinates of the i-th reference atom of the first protein monomer in the ground truth protein complex are [x1, y1, z1], and the coordinates of the second ground truth coordinates of the j-th reference atom of the second protein monomer are [x2, y2, z2], then the element value of the element at the i-th row and the j-th column of the ground truth distance map represents the distance between [x1, y1, z1] and [x2, y2, z2].

In step 120, input features corresponding to the current training data are obtained.

Each entry of current training data has its corresponding input features. The input features corresponding to the current training data are determined based on the first amino acid sequence, the second amino acid sequence, the monomeric 3D structural information of the first protein, and the monomeric 3D structural information of the second protein. In other words, the input features of the current training data reflect both protein sequence modality information and protein structural modality information, allowing for multi-dimensional characterization of proteins. Compared to using only single-modality information, using multi-modality information can obtain more accurate prediction results.

Exemplarily, the first amino acid sequence, the second amino acid sequence, the monomeric 3D structural information of the first protein, and the monomeric 3D structural information of the second protein in the current training data can be input into a trained feature extraction model to obtain the input features of the current training data; alternatively, preset feature extraction operations can be performed on the current training data to obtain the input features of the current data. Specific methods for determining input features can refer to prior arts of AlphaFold Multimer, etc.

In step 130, model prediction comprises: inputting the input features into a protein complex structure prediction model having current parameters to obtain a prediction result.

The protein complex structure prediction model has current parameters, which will be updated in subsequent steps based on the loss function.

Exemplarily, the protein complex structure prediction model in examples of the present disclosure includes a multi-layer perceptron (MLP) and an evolutionary module (also referred to herein as "evolutionary network module"). The multi-layer perceptron can be used to capture relationships between input features, and the evolutionary module iteratively evolves the features input into it to obtain evolved features. The structure of the evolutionary module can refer to the architecture of Evoformer in AlphaFold Multimer. Then, through a post-processing layer, the evolved features are processed to obtain a prediction result (the post-processing layer used to obtain the predicted distance map is also referred to herein as a "map generation module"). The multi-layer perceptron, evolutionary module, and post-processing layer contain learnable parameters. It can be understood that when the parameters of the protein complex structure prediction model change, the prediction result obtained by the protein complex structure prediction model with those parameters also changes accordingly.

The prediction result includes a predicted distance map. The predicted distance map and the ground truth distance map can have the same shape. Exemplarily, when the first protein and the second protein contain m and n amino acid residues respectively, the predicted distance map includes an m*n 2D matrix. An element at the i-th row and the j-th column (i=1..m, j=1..n) of the predicted distance map represents the predicted distance between the i-th reference atom of the first protein and the j-th reference atom of the second protein when the first protein and the second protein form a protein complex, as predicted by the protein complex structure prediction model. In other words, examples of the present disclosure do not directly predict the 3D structural information of the protein complex, but rather predict the distances between reference atoms located on different protein monomers when forming the protein complex. Correspondingly, the ground truth distance map includes an m*n 2D matrix, and an element at the i-th row and the j-th column (i=1..m, j=1..n) of the ground truth distance map represents the ground truth distance between the i-th reference atom of the first protein and the j-th reference atom of the second protein in the ground truth protein complex.

Exemplarily, the prediction result including the predicted distance map in examples of the present disclosure can be understood in a broad sense. The predicted distance map can be obtained by processing the prediction result output by the post-processing layer. For example, the post-processing layer outputs an (m+n) * (m+n) matrix, which contains four regions: m*m, n*n, m*n, and n*m. Among them, the matrix elements in the m*m region represent the distances between reference atoms in the first protein predicted by the model, the matrix elements in the n*n region represent the distances between reference atoms in the second protein predicted by the model, the matrix elements in the m*n region represent the distances between reference atoms in the first protein and reference atoms in the second protein predicted by the model, and the matrix elements in the n*m region represent the distances between reference atoms in the second protein and reference atoms in the first protein predicted by the model, wherein the values of the matrix elements in the m*n region and the n*m region should be symmetrical. Since only the distances between reference atoms of the first protein and reference atoms of the second protein are used subsequently, only the m*n region of the (m+n)*(m+n) matrix output by the post-processing layer can be selected as the predicted distance map.

Exemplarily, the element values at the i-th row and j-th column (i=1..m, j=1..n) of the predicted distance map and the ground truth distance map can be continuous distance values (in which case the distance map can be called a continuous distance map), or discrete distance categories (in which case the distance map can be called a discrete distance map). If both discrete distance maps and continuous distance maps are needed for subsequent loss function calculations, the predicted distance map can include a discrete predicted distance map and a continuous predicted distance map, and the ground truth distance map can also include a discrete ground truth distance map and a continuous ground truth distance map. The following describes how to obtain continuous ground truth distance maps and discrete ground truth distance maps. When calculating element values in the ground truth distance map, the first ground truth coordinates of the i-th reference atom of the first protein and the second ground truth coordinates of the j-th reference atom of the second protein can be found from the 3D structural information of the ground truth protein complex, and then the distance value between the two ground truth coordinates is calculated. The 2D matrix obtained by using this distance value as the element value at the i-th row and j-th column can be used as the continuous ground truth distance map. When determining the discrete ground truth distance map, first, a mapping relationship between distance values and distance categories is set. For example, a specified distance range can be divided into P categories (e.g., 64 categories), thereby obtaining a mapping relationship between distance ranges and distance categories, where distance values falling into a distance range correspond to the corresponding distance category. Then, based on the mapping relationship between the distance value at the i-th row and j-th column of the continuous ground truth distance map and the distance categories, the distance category corresponding to the distance value at the i-th row and j-th column is determined; finally, the distance category is used as the element value of the element at the i-th row and j-th column in the discrete ground truth distance map. Using distance categories instead of specific distance values is because the 3D structural information of the ground truth protein complex is determined by experimental methods, and due to experimental resolution, the distances directly calculated from the ground truth coordinates in the 3D structural information of the ground truth protein complex have noise. Directly using specific distance values to calculate distance loss values will introduce noise, and using distance categories instead of specific distance values can reduce the impact of noise.

Exemplarily, based on the element values of the discrete ground truth distance map representing distance categories, further, the element values of the discrete ground truth distance map can be a vector or a set of vectors used to represent distance categories. In this case, the element value at the i-th row and j-th column of the discrete ground truth distance map can be determined by the following steps: first, determine the distance category corresponding to the distance value between the i-th reference atom of the first protein and the j-th reference atom of the second protein by the method described above, and then determine the one-hot encoding vector (size 1*P) corresponding to that distance category, and use this vector as the element value at the i-th row and j-th column of the ground truth distance map. In this way, it facilitates subsequent calculation of the loss value between the predicted distance map and the ground truth distance map. Exemplarily, when the ground truth distance map used in the distance loss function is discrete, the predicted distance map used in the distance loss function is also discrete. In this case, the element at the i-th row and j-th column of the discrete predicted distance map is the distance category where the distance between the i-th reference atom of the first protein and the j-th reference atom of the second protein belongs when the first protein and the second protein form a protein complex, as predicted by the model. In other words, in this case, the prediction of the predicted distance map by the protein complex structure prediction model is a classification problem. It can be understood that the distance category predicted by the model can be represented by a vector (e.g., a 1*P vector, that is, the element at the i-th row and j-th column has P components, and the predicted distance map is an m*n*P tensor), where each component in the vector represents the probability that the distance between the i-th reference atom of the first protein and the j-th reference atom of the second protein belongs to each distance category.

Exemplarily, when the ground truth distance map includes both continuous and discrete versions, one can be derived from the other. For example, for the ground truth distance map, a continuous ground truth distance map is first calculated, and then a discrete ground truth distance map is derived from the continuous ground truth distance map, the specific process is described above. Similarly, when the predicted distance map includes both continuous and discrete versions, one can also be derived from the other. For example, for the predicted distance map, the protein complex structure prediction model outputs predicted values for the discrete predicted distance map (predicting which distance category the distance between the i-th atom of the first protein and the j-th reference atom of the second protein falls into), and a continuous predicted distance map needs to be derived from the discrete predicted distance map. In this case, the specific distance value corresponding to the center of the distance range corresponding to the distance category with the highest probability can be used as the distance value corresponding to the distance category. For example, if the distance range from 0 Å to 64 Å is equally divided into 64 categories to obtain 64 distance categories, the 23rd category corresponds to a distance range of 22 Å - 23 Å, and the specific distance value corresponding to the center of the 23rd category is 22.5 Å. If the 23rd component of the 1 * 64 vector corresponding to the i-th row and j-th column in the discrete predicted distance map is the largest, then the element value at the i-th row and j-th column in the continuous predicted distance map is 22.5.

In step 140, the current parameters are updated based on a loss function to train and obtain the protein complex structure prediction model (the trained protein complex structure prediction model is also referred to herein as "target map prediction network").

The distance loss function in the loss function (also referred to as "outer loss function" hereinafter) is used to represent the mapping relationship between the first difference and the parameters of the protein complex structure prediction model, i.e., the distance loss function takes the model parameters as independent variables and the first difference as the function value. It can be understood that the predicted distance map and the ground truth distance map used when calculating the distance loss function are discrete, and the element values in the discrete predicted distance map and the discrete ground truth distance map can represent distance categories. Specifically, the element value of the discrete predicted distance map can be a vector, where each element in the vector represents the probability that the predicted distance belongs to each distance category; the element value in the discrete ground truth distance map is also a vector, which is a one-hot encoding vector of the distance category. The element values of both the discrete predicted distance map and the discrete ground truth distance map include K components, where K is the total number of distance categories.

Exemplarily, when the total number of distance categories is K, the distance loss function $L_{dist}$ is in the form of formula (1).

$$L_{dist} = -\frac{1}{mn} \sum_{i,j} \sum_{k=1}^{K} \bar{G}_{ij}^k \log \bar{D}_{ij}^k \qquad (1)$$

wherein $\bar{G}_{ij}^k$ is the k-th component of the element value of the element at the i-th row and j-th column of the discrete ground truth distance map, and $\bar{D}_{ij}^k$ is the k-th component of the element value at the i-th row and j-th column of the discrete predicted distance map.

Exemplarily, the parameters of the protein complex structure prediction model can be updated multiple times using multiple samples until the training iterations are reached or the model converges, thereby obtaining a trained protein complex structure prediction model.

Exemplarily, a training data pool can be prepared. Each time, one data entry from the training data pool is taken as the current training data. After performing the steps included in the training method 100 based on the current training data, another training data entry is obtained from the training data pool as the current training data, i.e., the current training data is updated. For example, the training data pool includes 1000 training data entries, and the entire training process includes 5 epochs, with 1000 training data entries used in each epoch. In the first epoch, at each iteration, one unused training data entry is taken from the 1000 training data entries in the training data pool, and 1000 iterations are performed; in the second epoch, at each iteration, one unused training data entry is taken from the 1000 training data entries in the training data pool, and 1000 iterations are performed; ... and so on for 5 epochs, then training ends.

In step 140, the current parameters can be updated based on the gradient of the loss function with respect to the current parameters, using a conventional optimizer.

It can be seen that examples of the present disclosure provide sequence modality information and structural modality information of protein monomers to the protein complex structure prediction model, allowing the model to predict the predicted distance map using multi-modal information; at the same time, it effectively utilizes the rigid body characteristics of protein monomers, transforming the protein complex structure prediction problem into a rigid body docking problem, and then using a rigid body docking model to solve for the optimal docking pose that most closely matches the predicted distance map, thereby predicting the 3D structure of the protein complex. The complex structure prediction model in examples of the present disclosure does not directly predict the 3D structure of the protein complex, but rather predicts the distances between atoms when forming the protein complex. This allows for accurate prediction of protein complex docking relationships without using complex structure prediction models, and can improve the accuracy of prediction results while greatly reducing model complexity and computational consumption.

In a specific embodiment, the loss function further includes an inner loss function, wherein the inner loss function is configured to represent a mapping relationship between a second difference and an optimal docking pose; the second difference is a difference between distances between reference atoms included in each amino acid residue of the first protein and reference atoms included in each amino acid residue of the second protein when the first protein and the second protein are docked into a protein complex in the optimal docking pose, and the ground truth distance map; the method 100 further comprises:

In step 150, determining an optimal docking pose of the first protein and the second protein under the current parameters, wherein, under the current parameters, when the first protein and the second protein are docked into a protein complex in the optimal docking pose, a difference between distances between reference atoms included in each amino acid residue of the first protein and reference atoms included in each amino acid residue of the second protein and the predicted distance map is less than a difference when docked in a non-optimal docking pose.

A docking pose can be represented by a translation relationship t and a rotation relationship R. The following describes an example where the position and pose of the first protein remain fixed, and the second protein adjusts its position and pose with a docking pose to dock with the first protein to form a protein complex. The monomer three-dimensional structure of the first protein and the monomer three-dimensional structure of the second protein are known, it can be determined what kind of translation and rotation the second protein has undergone relative to the monomer three-dimensional structure of the second protein when docking based on the docking pose. Thus, the 3D structure of the second protein during docking, i.e., the coordinates of each reference atom in the second protein during docking, can be obtained. This allows for determining the distances between reference atoms of the first protein and reference atoms of the second protein when docked in a certain docking pose (also referred to herein as "calculated distance map"). It can be understood that when docked in different docking poses, the distances between reference atoms of the first protein and reference atoms of the second protein are different, and the differences from the element values in the predicted distance map are also different.

After the protein complex structure prediction model with current parameters predicts a predicted distance map, in the entire docking pose space, there exists a docking pose such that when the first protein and the second protein are docked into a protein complex in this docking pose, the difference between the distances between reference atoms included in each amino acid residue of the first protein and reference atoms included in each amino acid residue of the second protein and the predicted distance map is less than the difference between the distances between reference atoms included in each amino acid residue of the first protein and reference atoms included in each amino acid residue of the second protein when the first protein and the second protein are docked in other docking poses. In other words, when docked into a protein complex in this docking pose, the distances between reference atoms included in each amino acid residue of the first protein and reference atoms included in each amino acid residue of the second protein are closest to the predicted distance map. Such a docking pose is called an optimal docking pose. It can be understood that when the parameter $\Phi$ of the protein complex structure prediction model changes, the predicted distance map obtained by the protein complex structure prediction model with parameter $\Phi$ also changes accordingly, and the optimal docking pose solved based on the predicted distance map also changes accordingly. In other words, the predicted distance map is a function of the model parameter $\Phi$, and the optimal docking pose is also a function of the model parameter $\Phi$.

When the parameters of the protein complex structure prediction model are the current parameters $\Phi$, and the docking pose is R, t, the difference between the distances between each reference atoms of the first protein and each reference atoms of the second protein and the predicted distance map can be expressed by formula (2).

$$\mathcal{L}(R, \mathbf{t}, \phi) = \min_{R, \mathbf{t}} \frac{1}{mn} \sum_{i=1}^{m} \sum_{j=1}^{n} \left( \|X_i - RY_j - \mathbf{t}\| - \hat{D}_{ij}(\phi) \right)^2 \tag{2}$$

Wherein $X_i$ is the coordinates of the reference atom included in the i-th amino acid residue in the monomeric 3D structural information of the first protein, i=1,2,...m; $Y_j$ represents the coordinates of the reference atom included in the j-th amino acid residue in the monomeric 3D structural information of the second protein, j=1,2,...n. $\hat{D}$ represents the predicted distance map.

When the parameters of the protein complex structure prediction model are the current parameters $\Phi$, the optimal docking pose is the translation relationship t and rotation relationship R that minimize L(R,t,$\Phi$) in formula (2), hereinafter denoted as t* and R*.

$$R^*(\phi), \mathbf{t}^*(\phi) = \underset{R, \mathbf{t}}{\mathrm{argmin}} \, \mathcal{L}(R, \mathbf{t}, \phi)$$

Exemplarily, in addition to the distance loss function, the loss function also includes an inner loss function. In this case, the loss function can be a weighted sum of the distance loss function, and the inner loss function (or other loss functions). The inner loss function is configured to represent a mapping relationship between the second difference and the optimal docking pose, i.e., the inner loss function takes the optimal docking pose as an independent variable and the second difference as the function value. The second difference is the difference between the distances between reference atoms included in each amino acid residue of the first protein and reference atoms included in each amino acid residue of the second protein when the first protein and the second protein are docked into a protein complex in the optimal docking pose, and the ground truth distance map. Exemplarily, the inner loss function L$^{in}$ can be expressed by formula (3).

$$\mathcal{L}^{in}\left(R^*(\phi), \mathbf{t}^*(\phi)\right) = \frac{1}{mn} \sum_{i=1}^{m} \sum_{j=1}^{n} \left( \|X_i - R^*(\phi)Y_j - \mathbf{t}^*(\phi)\| - D_{ij} \right)^2 \tag{3}$$

Wherein $X_i$ is the coordinates of the reference atom included in the i-th amino acid residue in the monomeric 3D structural information of the first protein, i=1,2,...m; $Y_j$ represents the coordinates of the reference atom included in the j-th amino acid residue in the monomeric 3D structural information of the second protein, j=1,2,...n. D represents the ground truth distance map.

Exemplarily, the predicted distance map used in formula (2) and the ground truth distance map used in formula (3) can both be continuous. In this case, the predicted distance map has two versions, continuous and discrete, and the ground truth distance also has two versions, continuous and discrete, wherein the discrete predicted distance map and the discrete ground truth distance map are used to calculate L$_{dist}$, and the continuous predicted distance map and the continuous ground truth distance map are used to calculate t* and R*, L$^{in}$, etc.

Exemplarily, the predicted distance map and the ground truth distance map can both include discrete and continuous versions. The predicted distance map predicted by the protein complex structure prediction model is a discrete predicted distance map (also referred to herein as "quasi-predicted distance map"), and a continuous predicted distance map can be calculated based on the discrete predicted distance map; a continuous ground truth distance map can be calculated from the 3D structural information of the ground truth protein complex composed of the first protein and the second protein, and then the continuous ground truth distance map can be discretized into a discrete distance map.

Exemplarily, step 150 is performed before step 140. In step 150, the optimal docking pose is calculated. In step 140, the gradient value of the loss function under the current parameters is calculated based on the optimal docking pose, and the current parameters are updated using this gradient value.

Examples of the present disclosure incorporate an inner loss function reflecting the docking result into the loss function, which can adjust model parameters based on the difference between the optimal docking result obtained under current parameters and the ground truth value, enabling the model to take the improvement of the optimal docking result as the direct optimization goal, thereby improving model training efficiency and effectiveness.

In a specific embodiment, step 150 comprises: based on an initial value of the docking pose, iteratively updating the initial value of the docking pose through T times of gradient descent based on a difference between distances between reference atoms included in each amino acid residue of the first protein and reference atoms included in each amino acid residue of the second protein and the predicted distance map when the first protein and the second protein are docked into a protein complex in the current docking pose, to obtain an approximate solution of the optimal docking pose.

As described above, the inner loss function L$^{in}$ is a function of the optimal docking poses R* and t*, and the optimal docking poses R* and t* are functions of the current parameters, and the distance loss function is a function of the current parameters. Therefore, optimizing the current parameters and solving for the optimal docking pose under the current parameters form a nested structure, and the model training problem is transformed into a two-layer optimization problem that can be expressed by formula (4).

$$\phi^* = \underset{\phi}{\mathrm{argmin}}\ \mathcal{L}^{out}\left(R^*(\phi), \mathbf{t}^*(\phi), \phi\right) \quad \text{s.t.}\ R^*(\phi), \mathbf{t}^*(\phi) = \underset{R,\mathbf{t}}{\mathrm{argmin}}\ \mathcal{L}(R, \mathbf{t}, \phi)$$

$$(4)$$

Wherein the outer loop is used to update the model parameters of the protein complex structure prediction model, and the inner loop is dedicated to solving the optimal docking pose problem, where $L^{out}$ is the loss function.

To solve the two-layer optimization problem, the inner loop is approximated by T times of gradient descent (T needs to be large enough to obtain an approximate solution sufficiently close to the optimal docking poses R* and t*, for example, T is 2000), i.e., R and t obtained through T times of gradient descent are used as approximate solutions for the optimal docking poses R* and t*. Exemplarily, in the (t+1)-th gradient descent, the docking pose (represented by translation relationship t and rotation relationship **R,** it should be noted that t representing translation relationship has directionality and needs to be distinguished from t representing the number of gradient descent iterations) is iteratively updated as shown in formula (5).

$$\mathbf{R}_{t+1} = \mathrm{OPT}(\mathbf{R}_t, \nabla_{\mathrm{R}} L_t\ (\mathbf{R}_t, \mathbf{t}_t, \Phi));$$

$$\mathbf{t}_{t+1} = \mathrm{OPT}(\mathbf{t}_t, \nabla_t L_t(\mathbf{R}_t, \mathbf{t}_t, \Phi));$$

$$(5)$$

wherein, t=0, 1, 2, ... T-1. OPT represents an optimization algorithm, such as Stochastic gradient descent (SGD) or ADAM algorithm. $\nabla_{\mathrm{R}} L_t$ is the gradient of formula (2) with respect to R in the (t+1)-th gradient descent, and $\nabla_t L_t$ is the gradient of formula (2) with respect to t in the (t+1)-th gradient descent.

$R_0$ and $t_0$ can be random initial values or initial values determined by other means.

Exemplarily, since the orthogonality of the rotation matrix representing the rotation relationship **R** cannot be guaranteed, **R** is converted to an orthogonal vector **q**, and $\nabla_{\mathbf{q}} L_t(\mathbf{R}_t, \mathbf{t}_t, \Phi)$ is used instead of $\nabla_{\mathbf{R}} L_t(\mathbf{R}_t, \mathbf{t}_t, \Phi)$.

$\nabla_{\mathbf{q}} L_t(\mathbf{R}_t, \mathbf{t}_t, \Phi)$ and $\nabla_t L_t(\mathbf{R}_t, \mathbf{t}_t, \Phi)$ are determined by formula (6):

$$\tilde{D}_{ij} = X_i - R_t Y_j - \mathbf{t}_t,$$

$$\nabla_{\mathbf{q}} \mathcal{L}_t\left(R_t, \mathbf{t}_t, \phi\right) = \sum_{i,j} \frac{2}{mn} \frac{\hat{D}_{ij} - \|\tilde{D}_{ij}\|}{\|\tilde{D}_{ij}\|} \tilde{D}_{ij} \odot Y_j \cdot \frac{\partial R_t}{\partial \mathbf{q}_t},$$

$$\nabla_{\mathbf{t}} \mathcal{L}_t\left(R_t, \mathbf{t}_t, \phi\right) = \sum_{i,j} \frac{2}{mn} \frac{\hat{D}_{ij} - \|\tilde{D}_{ij}\|}{\|\tilde{D}_{ij}\|} \tilde{D}_{ij},$$

$$(6)$$

wherein $\mathbf{q}_t$ is the orthogonal vector obtained by matrix transformation of $\mathbf{R}_t$; $X_i$ is the coordinates of the i-th reference atom in the monomeric 3D structure of the first protein, $Y_j$ is the coordinates of the j-th reference atom in the monomeric 3D structure of the second protein, $\mathbf{R}_t$ is the rotation relationship updated by the t-th gradient descent, $\mathbf{t}_t$ is the translation relationship updated by the t-th gradient descent, $\tilde{D}_{ij}$ is the distance between the i-th reference atom of the first protein and the j-th reference atom of the second protein in the protein complex formed by docking the first protein monomer and the second protein monomer with docking poses $\mathbf{R}_t$ and tt; $\hat{D}_{ij}$ is the element at the i-th row and j-th column of the predicted distance map. The element values of $\tilde{D}_{ij}$ and $\hat{D}_{ij}$ pred can be specific distance values, i.e., the predicted distance map when calculating $\mathbf{R}^*$ and $\mathbf{t}^*$ is continuous. $\odot$ 0epresents the Hadamard product.

When performing outer loop optimization, i.e., updating the current parameters using the loss function, the differential expression of the loss function $L^{out}$ with respect to the current parameter $\Phi$ can be expressed by formula (7).

$$d_\phi \mathcal{L}^{out} = \frac{\partial \mathcal{L}^{out}}{\partial (R^*, \mathbf{t}^*)} \frac{\partial (R^*(\phi), \mathbf{t}^*(\phi))}{\partial \phi} + \frac{\partial \mathcal{L}^{out}}{\partial \phi}. \tag{7}$$

After approximating $R^*$ and $\mathbf{t}^*$ as $R_T$ and $\mathbf{t}_T$, some terms in formula (7) can be transformed as shown in formula (8). The term $\frac{\partial \mathcal{L}^{out}}{\partial (R^*, \mathbf{t}^*)}$ in formula (7) can be calculated according to formula (3), and the term $\frac{\partial \mathcal{L}^{out}}{\partial \phi}$ in formula (7) can be calculated according to formula (1), thereby calculating $d_\phi L^{ou}$ for updating the model parameter $\Phi$.

$$\frac{\partial (R^*(\phi), \mathbf{t}^*(\phi))}{\partial \phi} \approx -\gamma \frac{\partial^2 \mathcal{L}(R_T, \mathbf{t}_T, \phi)}{\partial (R_T, \mathbf{t}_T)^\top \partial \phi} \tag{8}$$

In a specific embodiment, the initial values of the docking poses of the first protein and the second protein are determined by a spectral initialization method.

Due to the complexity of the optimization problem and the rugged optimization landscape, gradient descent in the inner loop often requires a large number of iterations to converge and faces the challenge of finding a global minimum. Therefore, in a specific example, before starting iterative optimization, a numerical solution for $R$ and $\mathbf{t}$ is obtained through a spectral initialization method, providing a more favorable starting point for the convergence of the inner optimization problem.

Let $\hat{Y} = RY - \mathbf{t}\mathbf{1}_n^\top$, where $\mathbf{1}_n$ is an all-ones vector. Then, use formula (9) instead Y.

$$\left( \|X_i - RY_j - \mathbf{t}\| - \hat{D}_{ij} \right)^2 = X_i^\top X_i - 2X_i^\top \hat{Y}_j + \hat{Y}_j^\top \hat{Y}_j + \hat{D}_{ij}^2 - 2\hat{D}_{ij}\|X_i - \hat{Y}_j\| \tag{9}$$

Based on these variable combinations, we define four variables and two central matrices as shown in formula (10).

$$B = \begin{bmatrix} X_1^\top X_1 & \dots & X_1^\top X_1 \\ X_2^\top X_2 & \dots & X_2^\top X_2 \\ \dots & \dots & \dots \\ X_m^\top X_m & \dots & X_m^\top X_m \end{bmatrix}_{m \times n} \quad C = \begin{bmatrix} \hat{Y}_1^\top \hat{Y}_1 & \dots & \hat{Y}_n^\top \hat{Y}_n \\ \hat{Y}_1^\top \hat{Y}_1 & \dots & \hat{Y}_n^\top \hat{Y}_n \\ \dots & \dots & \dots \\ \hat{Y}_1^\top \hat{Y}_1 & \dots & \hat{Y}_n^\top \hat{Y}_n \end{bmatrix}_{m \times n}$$

$$E = X^\top \hat{Y} = \begin{bmatrix} X_1^\top \hat{Y}_1 & \dots & X_1^\top \hat{Y}_n \\ X_2^\top \hat{Y}_1 & \dots & X_2^\top \hat{Y}_n \\ \dots & \dots & \dots \\ X_m^\top \hat{Y}_1 & \dots & X_m^\top \hat{Y}_n \end{bmatrix}_{m \times n} \quad F = \begin{bmatrix} \hat{D}_{11}^2 & \dots & \hat{D}_{1n}^2 \\ \hat{D}_{21}^2 & \dots & \hat{D}_{2n}^2 \\ \dots & \dots & \dots \\ \hat{D}_{m1}^2 & \dots & \hat{D}_{mn}^2 \end{bmatrix}_{m \times n}$$

$$H_m = I_m - \frac{1}{m} J_m \quad H_n = I_n - \frac{1}{n} J_n, \tag{10}$$

Wherein I represents the identity matrix, and J represents the 1s matrix. If the condition $\forall i,j, \|X_i - \hat{Y}_j\| = \hat{D}_{ij}$ holds, then there exists an equality as shown in formula (11):

$$H_m F H_n = H_m (B - 2E + C) H_n = -2 H_m E H_n = -2 H_m X^\top R Y H_n. \tag{11}$$

Perform singular value decomposition (SVD) on $H_m X^\top$ and $Y H_n$ respectively as shown in formula (12),

$$H_m X^\top = U_X \Sigma_X V_X^\top, \quad Y H_n = U_Y \Sigma_Y V_Y^\top, \tag{12}$$

the solution for the rotation relationship can be derived as shown in formula (13), and the R solved by formula (13) is used as $R_0$.

$$R = -\frac{1}{2} V_X \Sigma_X^{-1} U_X^\top H_m F H_n V_Y \Sigma_Y^{-1} U_Y^\top \qquad (13)$$

Given the rotation relationship **R**, the translation relationship t is solved using formula (14),

$$H_m F = H_m (B - 2E + C) = H_m B - 2H_m X^\top R Y - 2H_m X^\top t 1_n^\top \qquad (14)$$

The solution for t is shown in formula (15), and the t solved by formula (15) is used as $\mathbf{t}_0$.

$$\mathbf{t} = -\frac{1}{2} V_X \Sigma_X^{-1} U_X^\top H_m (F - B + 2X^\top \hat{R} Y) 1_n \qquad (15)$$

By using the values obtained from the above spectral initialization instead of random initial values as $\mathbf{R}_0$ and $\mathbf{t}_0$, gradient descent will approach the global optimal solution more efficiently and accurately.

In a specific embodiment, step 120 comprises: determining input features of the current training data.

Wherein, the input features include sequence modality features and structural modality features. The sequence modality features are determined based on the first amino acid sequence and the second amino acid sequence, and mainly include information obtained from the sequences themselves. The sequence modality features include type features $F^{typ}$, sequence pairing features $F^{pp}$, and may also include multiple sequence features $F^{msa}$. Among them, the type features $F^{typ}$ are used to characterize the type of amino acid at each position in the first amino acid sequence and the second amino acid sequence; the sequence pairing features $F^{pp}$ are used to characterize the positional relationship between amino acids. The structural modality features are determined based on the monomeric 3D structural information of the first protein and the monomeric 3D structural information of the second protein, and include structural pairing features $F^p$ and angle features $F^{ang}$. The structural pairing features $F^p$ are used to characterize the spatial distance between reference atoms; the angle features $F^{ang}$ are used to characterize the torsion angles of the main chain backbone and side chains of the first protein and the second protein.

The acquisition of sequence modality features and structural modality features can refer to the description in AlphaFold Multimer, and the following is only an exemplary description.

When extracting sequence modality features and structural modality features, the first protein and the second protein can be concatenated (specific connection symbols can be used in between) for calculation, where Nres represents the number of amino acids after concatenation.

Exemplarily, type features $F^{typ} \in R^{Nres \times 21}$ include one-hot representations of amino acid types, where amino acid types include 20 known amino acids and one unknown type.

Exemplarily, sequence pairing features $F^{pp} \in R^{Nres \times Nres \times 73}$ contain positional information within or between chains, including the following three components: (1) Relative positional features of size $[N_{res}, N_{res}, 66]$, used to represent relative amino acid residue indices, which are mapped to [-32, 32]. The 66th index is used to indicate that two amino acids are located on different proteins. (2) An indicator of size $[N_{res}, N_{res}, 1]$ indicating whether two amino acid residues are derived from the same protein monomer. (3) Relative index features of size $[N_{res}, N_{res}, 6]$, wherein the first 5 components of the relative index features indicate relative sym_id indices mapped to [-2, 2], and the 6th component is used to indicate pairs of two residues containing different sym_ids, specifically referring to the description in AlphaFold Multimer.

Angle features $F^{ang}$ can be jointly obtained from the monomeric 3D structural information of the first protein and the monomeric 3D structural information of the second protein. Exemplarily, angle features $F^{ang} \in R^{Nres \times 57}$ include three components. (1) One-hot representation of amino acid types of size $[N_{res}, 22]$, including 20 amino acids, one unknown type, and one gap or missing residue. (2) Angle representation of size $[N_{res}, 28]$, which uses sine and cosine to encode three backbone torsion angles, four side chain torsion angles, and an optional torsion angle with 180 rotational symmetry for each residue local framework. (3) Angle indicators of size $[N_{res}, 7]$, indicating the presence or absence of torsion angles.

Structural pairing features $F^p \in R^{Nres \times Nres \times 88}$ contain five components. (1) Binary graph features of size $[N_{res}, N_{res}, 39]$ representing discrete distances between Cβ atoms. In the absence of Cβ atoms in glycine, Cα atoms are used instead. Distances are discretized into 38 equally spaced categories, ranging from 3.25 Å to 50.75 Å, with one category representing distances greater than 50.75 Å. (2) Residue type features of size $[N_{res}, N_{res}, 44]$ are extended from one-hot representations of residue types, with sizes $[N_{res}, 1, 22]$ and $[N_{res}, 22, 1]$. (3) Backbone features of size $[N_{res}, N_{res}, 3]$ are obtained by constructing unit vectors of local backbones based on original N-Cα-C coordinates using the Gram-Schmidt process. (4) Residue indicators of size $[N_{res}, N_{res}, 1]$ are extended from indicators of residue presence. (5) Pair indicators of size $[N_{res}, N_{res}, 1]$ indicate whether the pair is masked.

Exemplarily, the protein complex structure prediction model includes a multi-layer perceptron and an evolutionary module, and may also include a post-processing layer. The current parameters include parameters of the multi-layer perceptron and parameters of the evolutionary module, and may also include parameters of the post-processing layer. The process of inputting the input features into the protein complex structure prediction model with current parameters to obtain a prediction result is shown in FIG.3. Exemplarily, step 130 includes:

Step a, processing sequence pairing features and structural pairing features using a multi-layer perceptron to obtain integrated first features.

Exemplarily, the sequence pairing feature $F^{pp}$ is processed by a first multi-layer perceptron to obtain a transformed sequence pairing feature $P^{pp}$ ; and the structure pairing feature $F^p$ is processed by a second multi-layer perceptron to obtain a transformed structure pairing feature $P^p$.

The transformed sequence pairing features and the transformed structural pairing features are added to obtain integrated first features P, as shown in formula (16).

$$P = P^{pp} + P^p \qquad (16)$$

Step b, processing type features, multiple sequence features, and angle features using a multi-layer perceptron to obtain integrated second features;

Exemplarily, the third multi-layer perceptron is used to process the type features $F^{typ}$ to obtain transformed type features $M^{typ}$; the fourth multi-layer perceptron is used to process the multiple sequence features $F^{msa}$ to obtain transformed multiple sequence features $M^{msa}$; the fifth multi-layer perceptron is used to process the angle features $F^{ang}$ to obtain transformed angle features $M^{ang}$.

The transformed type features and the transformed multiple sequence features are added, and then concatenated with the transformed angle features to obtain integrated second features M, as shown in formula (17).

$$M = \left[ (M^{typ} + M^{msa}) \| M^{ang} \right] \qquad (17)$$

Step c, inputting the integrated first features P and the integrated second features M into an evolutionary module evoformer to obtain evolved first features $\hat{P}$ and evolved second features $\hat{M}$;

Exemplarily, the integrated first features and the integrated second features can be input into the evolutionary module for multiple iterations to obtain evolved first features $\hat{P}$ and evolved second features $\hat{M}$ after multiple evolutions.

Step d, determining the predicted distance map based on the evolved first features $\hat{P}$.

Through a post-processing layer, non-linear transformation is performed on the evolved first features to obtain the predicted distance map.

Exemplarily, the evolved first features are $\hat{P}$, and through a post-processing layer, non-linear transformation as shown in formula (18) is performed on the evolved first features to obtain the predicted distance map $\bar{D}_{ij}$:

$$\bar{D}_{ij} = \sigma(W(\hat{P}_{ij} + \hat{P}_{ji})) \qquad (18)$$

wherein W is a learnable parameter in the post-processing layer, and $\sigma$ is an activation function.

It can be understood that, in this case, the current parameters also include W.

In a specific embodiment, the sequence modality features further include multiple sequence features. The multiple sequence features $F^{msa}$ are used to characterize the amino acid types of the multiple sequence alignment results of the first amino acid sequence and the second amino acid sequence, and can also characterize the amino acid masking situation (masking is also referred to herein as "covering").

Exemplarily, multiple sequence features $F^{MSA} \in R^{N_{cls} \times N_{res} \times 49}$ consist of five components ($N_{cls}$ represents the number of multi-sequence clustering centers, i.e. MSA clusters, also denoted as $N_{cluster}$): (1) One-hot representation of amino acid types of size [$N_{cluster}$, $N_{res}$, 23], including 20 amino acids, one unknown type, one gap or missing residue, and one mask token. (2) Amino acid distribution representation of size [$N_{cluster}$, $N_{res}$, 23], used to characterize the distribution of amino acid types within each MSA cluster. (3) Mask indicator of size [$N_{cluster}$, $N_{res}$, 1], indicating whether a mask exists to the left of each residue. (4) Mask value of size [$N_{cluster}$, $N_{res}$, 1] calculated using the formula $\frac{2}{\pi}\arctan\frac{3}{c}$ , wherein c represents the number of masks to the left of each position. (5) Average mask value of size [$N_{cluster}$, $N_{res}$, 1] calculated as $\frac{2}{\pi}\arctan\frac{3}{c}$ , wherein c' represents the average number of masks to the left of each position.

Multiple sequence features are obtained through the following steps: performing multiple sequence alignment on the first amino acid sequence and the second amino acid sequence to obtain multiple sequence alignment results, performing a masking operation on the multiple sequence alignment results (the multiple sequence alignment results are multiple amino acid sequences, and the masking operation refers to randomly masking a certain proportion of amino acids in the amino acid sequences, wherein the masked amino acids are replaced by mask tokens), and performing feature extraction on the results of the masking operation to obtain multiple sequence features. In this case, the prediction result further comprises reconstructing multiple sequences, where masked amino acids are predicted in the reconstructed multiple sequence.

In addition to steps a to d, step 130 further includes:

Step e, determining a reconstructed multiple sequence based on the evolved second features; exemplarily, the evolved second features are $\hat{M}$, and step e includes: performing linear transformation and softmax classification on $\hat{M}$ through a post-processing layer to obtain a reconstructed multiple sequence M. It can be understood that, in this case, the current parameters also include the parameters used for when transforming $\hat{M}$ in the post-processing layer.

Correspondingly, the loss function $L^{out}$ includes a distance loss function $L_{dist}$ an inner loss function $L^{in}$, and a reconstruction loss function $L_{msa'}$. For example, the loss function $L^{out}$ can be expressed by formula (19):

$$\mathcal{L}^{out} = \lambda_1 \mathcal{L}_{dist} + \lambda_2 \mathcal{L}_{msa} + \lambda_3 \mathcal{L}^{in}, \tag{19}$$

It can be understood that different model parameters lead to different predicted reconstructed multiple sequence M and different reconstruction losses. It can be seen that the reconstruction loss is related to the model parameters. The reconstruction loss function is used to represent the mapping relationship between the difference between the reconstructed multiple sequence and the multiple sequence alignment results and the parameters of the protein complex structure prediction model. Specifically, the reconstruction loss $L_{msa}$ can be expressed by formula (20):

$$\mathcal{L}_{msa} = -\frac{1}{N_{mask}} \sum_{i=1}^{N_{cls}} \sum_{j \in N_{mask}} \sum_{k=1}^{23} A_{ij}^k \log(\bar{M}_{ij}^k), \tag{20}$$

wherein, $N_{mask}$ is the number of masked amino acids (the masking ratio and method can be preset), A is the one-hot encoding of the ground truth amino acid type of the masked amino acid, and $\bar{M}_{ij}^k$ represents the k-th component of the predicted value at the i-th row and j-th column.

Similar to the prediction of the predicted distance map, the prediction of masked amino acids can also be regarded as a classification problem, it is predicted that the masked amino acid is one of multiple (for example, 23 types, corresponding to 20 amino acids, one unknown type amino acid, one gap or missing residue, and one mask token) candidate amino acids. The prediction result is represented by probabilities over 23 categories, wherein the k-th component of the prediction result represents the probability of predicting that the masked amino acid is the k-th amino acid among the 23 candidate amino acids. The ground truth value of the amino acid type of the masked amino acid is represented by the one-hot encoding of the category of the masked amino acid. For example, if the ground truth type of the masked amino acid is the 10th type, then in the one-hot encoding used as the ground truth value for that masked amino acid, the 10th component is 1, and the 1st-9th, 11th-23rd components are 0.

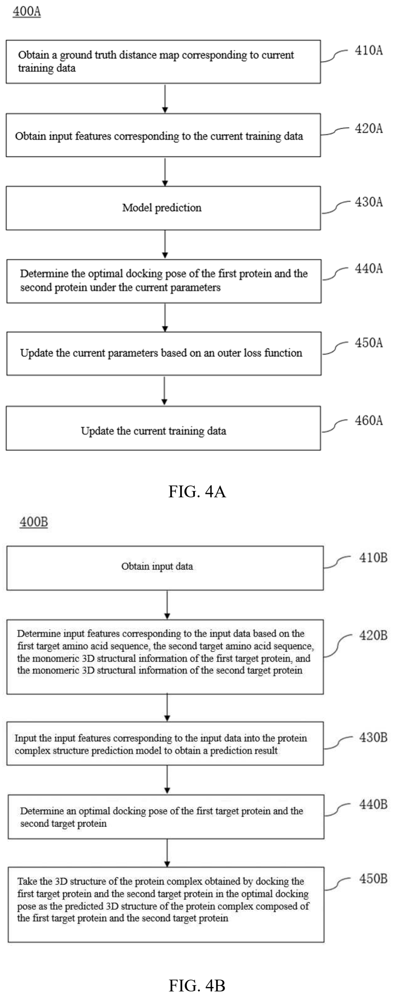

In a specific embodiment of the present disclosure, as shown in FIG. 4, another method 400A for training a protein complex structure prediction model includes steps 410A1 to 460A.

Step 410A: obtaining a ground truth distance map corresponding to current training data; wherein the current training data comprises a first amino acid sequence of a first protein, a second amino acid sequence of a second protein, monomeric three-dimensional (3D) structural information of the first protein, monomeric 3D structural information of the second protein, and 3D structural information of a ground truth protein complex composed of the first protein and the second protein; wherein the monomeric 3D structural information of the first protein comprises coordinates of reference atoms included in each amino acid residue thereof; the monomeric 3D structural information of the second protein comprises coordinates of reference atoms included in each amino acid residue thereof; the 3D structural information of the protein complex comprises first ground truth coordinates of reference atoms included in each amino acid residue of the first protein and second ground truth coordinates of reference atoms included in each amino acid residue of the second protein in the ground truth protein complex; and the ground truth distance map is determined based on the first ground truth coordinates and the second ground truth coordinates;

Step 420A: obtaining input features corresponding to the current training data; wherein the input features are determined based on the first amino acid sequence, the second amino acid sequence, the monomeric 3D structural information of the first protein, and the monomeric 3D structural information of the second protein;

Step 430A: performing model prediction, comprising: inputting the input features into a protein complex structure prediction model having current parameters to obtain a prediction result; wherein the prediction result comprises a predicted distance map; the predicted distance map and the ground truth distance map is two-dimensional (2D) matrices, wherein an element at an i-th row and a j-th column of the predicted distance map represents a distance between an i-th reference atom of the first protein and a j-th reference atom of the second protein when the first protein and the second protein form a protein complex, as predicted by the protein complex structure prediction model; and an element at the i-th row and the j-th column of the ground truth distance map represents a ground truth distance between the i-th reference atom of the first protein and the j-th reference atom of the second protein in the ground truth protein complex;

Step 440A: determining the optimal docking pose of the first protein and the second protein under the current parameters, wherein when the first protein and the second protein are docked into a protein complex in the optimal docking pose, distances between reference atoms included in each amino acid residue of the first protein and reference atoms included in each amino acid residue of the second protein are closest to the predicted distance map;

Step 450A: updating the current parameters based on an outer loss function; wherein the outer loss function comprises a weighted sum of a distance loss function and an inner loss function when the docking pose is the optimal docking pose; the distance loss function is configured to represent a mapping relationship between a first difference and the parameters of the protein complex structure prediction model, and the first difference is a difference between the predicted distance map and the ground truth distance map; the inner loss function is configured to represent a mapping relationship between a second difference and the parameters of the protein complex structure prediction model and the docking pose; and the second difference is a difference between distances between reference atoms included in each amino acid residue of the first protein and reference atoms included in each amino acid residue of the second protein when the first protein and the second protein are docked into a protein complex in the docking pose, and the predicted distance map;

Step 460A: updating the current training data;

The steps of obtaining the ground truth distance map corresponding to the current training data, obtaining the input features corresponding to the current training data, performing model prediction, determining the optimal docking pose of the first protein and the second protein under the current parameters, updating the current parameters based on the outer loss function, and updating the current training data are repeatedly executed until the number of iterations reaches a threshold of iterations or the protein complex structure prediction model converges, thereby obtaining a trained protein complex structure prediction model;

wherein, the initial values of the current parameters are initial parameters of the protein complex structure prediction model.

Exemplarily, step 440A comprises: based on an initial value of the docking pose, iteratively updating the initial value of the docking pose through T times of gradient descent based on the inner loss function when the parameters of the protein complex structure prediction model are the current parameters, to obtain an approximate solution of the optimal docking pose. Exemplarily, the initial value of the docking pose is determined based on a spectral initialization method.

Exemplarily, the input features in step 420A include sequence modality features and structural modality features; the sequence modality features comprise type features and sequence pairing features, wherein the sequence modality features are determined based on the first amino acid sequence and the second amino acid sequence; the type features are used to characterize amino acid types at each position in the first amino acid sequence and the second amino acid sequence; the sequence pairing features are used to characterize positional relationships between amino acids on the first amino acid sequence and the second amino acid sequence; the structural modality features comprise structural pairing features and angle features; wherein the structural modality features are determined based on the monomeric 3D structural information of the first protein and the monomeric 3D structural information of the second protein; the structural pairing features are used to characterize distances between amino acids on the first amino acid sequence and the second amino acid sequence; the angle features are used to characterize torsion angles of a main chain backbone and side chains of the first protein and the second protein;

Exemplarily, the protein complex structure prediction model includes a multi-layer perceptron and an evolutionary module; step 430A comprises:

Step 430A1: processing sequence pairing features and the structural pairing features according to the multi-layer perceptron to obtain integrated first features;

Step 430A2: processing the type features and the angle features according to the multi-layer perceptron to obtain integrated second features;

Step 430A3: inputting the integrated first features and the integrated second features into the evolutionary module to obtain evolved first features and evolved second features;

Step 430A4: determining the predicted distance map based on the evolved first features.

Exemplarily, the input features in step 420A further include multiple sequence features; the multiple sequence features are determined as follows: comprising performing multiple sequence alignment on the first amino acid sequence and the second amino acid sequence to obtain multiple sequence alignment results, performing a masking operation on the multiple sequence alignment results, and performing feature extraction on results of the masking operation to obtain the multiple sequence features; the multiple sequence features are used to characterize amino acid types and amino acid deletion status in the multiple sequence alignment results of the first amino acid sequence and the second amino acid sequence; step 430A2 comprises: processing type features, multiple sequence features, and angle features using the multi-layer perceptron to obtain integrated second features.

Exemplarily, the prediction result in step 430A further includes reconstructing multiple sequences); step 430A further comprises: determining the reconstructed multiple sequences based on the evolved second features;

wherein, the outer loss function includes a weighted sum of a distance loss function, an inner loss function, and a reconstruction loss function; the reconstruction loss function is configured to represent a mapping relationship between a difference between the reconstructed multiple sequences and the multiple sequence alignment results and the parameters of the protein complex structure prediction model.

For detailed descriptions of Steps 410A-450A, refer to the descriptions of Steps 110-150, which will not be repeated here.

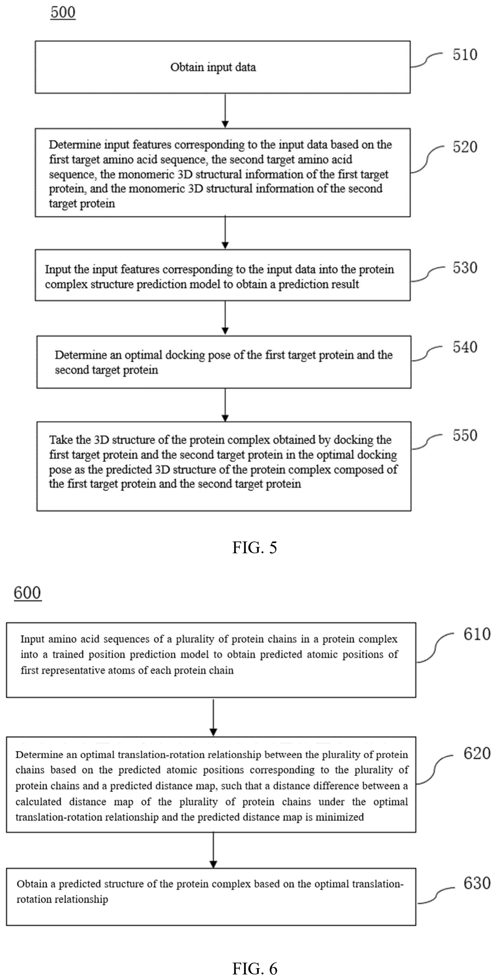

FIG. 5 is a flowchart of a method 500 for protein complex structure prediction according to an example of the present disclosure. As shown in FIG. 5, the method 500 includes steps 510 to 550.

Step 510, obtaining input data, wherein the input data comprises a first target amino acid sequence of a first target protein, a second target amino acid sequence of a second target protein, monomeric 3D structural information of the first target protein, and monomeric 3D structural information of the second target protein. The monomeric 3D structural information of the first target protein comprises coordinates of reference atoms included in each amino acid residue of the first target protein; the monomeric 3D structural information of the second target protein comprises coordinates of reference atoms included in each amino acid residue of the second target protein.

The first target protein and the second target protein are proteins that form a protein complex. The first target amino acid sequence and the second target amino acid sequence need to be known, and the monomeric 3D structural information of the first target protein and the monomeric 3D structural information of the second target protein also need to be known. If the monomeric 3D structural information of the first target protein and the monomeric 3D structural information of the second target protein are unknown, existing protein monomer structure prediction models (also referred to as "trained position prediction models" hereinafter) such as AlphaFold, Openfold, etc., can be used to predict the monomeric 3D structural information of the target proteins. If the first target protein or the second target protein is a chain in an antibody, existing antibody structure prediction models such as xTrimoABFold can be used to predict its 3D structural information.

It can be understood that, similar to the training phase, in the inference phase, for ease of calculation, for the convenience of calculation, the coordinates of the first target protein centroid can be transformed to the coordinate origin, and the coordinates of the second target protein centroid can also be transformed to the coordinate origin.

Step 520, determining input features corresponding to the input data based on the first target amino acid sequence, the second target amino acid sequence, the monomeric 3D structural information of the first target protein, and the monomeric 3D structural information of the second target protein.

The method for extracting input features corresponding to the input data is the same as the method for extracting input features of the current training data described in step 120, and is not repeated here.

Step 530, inputting the input features corresponding to the input data into the protein complex structure prediction model trained by the method 100 to obtain a prediction result; wherein the prediction result comprises a predicted distance map; the predicted distance map comprises a 2D matrix, wherein an element at the i-th row and the j-th column of the predicted distance map represents a predicted distance between the i-th reference atom of the first target protein and the j-th reference atom of the second target protein when the first target protein and the second target protein form a protein complex, as predicted by the protein complex structure prediction model.

For description of step 530, refer to the description of step 130, which will not be repeated here.

Exemplarily, the predicted distance map obtained in step 530 can be a discrete predicted distance map, where each element of the discrete predicted distance map is a 1*P vector, and each component in the vector represents the probability that the distance between the i-th reference atom of the first protein and the j-th reference atom of the second protein belongs to each distance category, as predicted by the model.

Step 540, determining an optimal docking pose of the first target protein and the second target protein, wherein when the first target protein and the second target protein are docked into a protein complex in the optimal docking pose, the difference between distances between reference atoms included in each amino acid residue of the first target protein and reference atoms included in each amino acid residue of the second target protein and the target predicted distance map is less than a difference when docked in a non-optimal docking pose. Wherein, the target predicted distance map is determined based on the predicted distance map.

It can be understood that the optimal docking pose is R and t that minimize formula (2). It should be noted that in step 540, $X_i$ is the coordinates of the reference atom included in the i-th amino acid residue in the monomeric 3D structural information of the first protein, $Y_j$ is the coordinates of the reference atom included in the j-th amino acid residue in the monomeric 3D structural information of the second protein. D is the predicted distance map. The model parameter $\Phi$ is fixed after training is completed.

When the first target protein and the second target protein contain m and n amino acids respectively, the target predicted distance map is an m*n 2D matrix, and the element value of each element in the matrix is a specific numerical value. The target predicted distance matrix is determined from the predicted distance map obtained in step 530 according to certain rules. These rules can be: for the element at the i-th row and j-th column in the predicted distance map, select a component that satisfies certain conditions from its corresponding vector (e.g., select one component from the first N components greater than a threshold M, wherein the selection can be random or according to preset rules), and use the distance corresponding to that component (i.e., the center of the distance range corresponding to the distance category corresponding to the component) as the element value of the element at the i-th row and j-th column in the target predicted distance map. For example, for the element at the i-th row and j-th column in the predicted distance map, if the k-th component in its corresponding vector is the largest, then the distance corresponding to the k-th component is used as the element value of the element at the i-th row and j-th column in the target predicted distance map (in this case, the threshold M is 0, N is 1). Another example: for the element at the i-th row and j-th column in the predicted distance map, the distance corresponding to the second largest component in its corresponding vector is used as the element value of the element at the i-th row and j-th column in the target predicted distance map (in this case, the threshold M is 0, N is 2, and the selection rule is to select the second largest). Yet another example: for the element at the i-th row and j-th column in the predicted distance map, randomly select one component from the top 3 largest components in its corresponding vector (in this case, the threshold M is 0, N is 3, and the selection is random), and use the distance corresponding to it as the element value of the element at the i-th row and j-th column in the target predicted distance map. In this way, multiple target predicted distance maps can be generated from one predicted distance map, and thus multiple optimal docking poses can be generated, leading to multiple different conformations of the protein complex.

Exemplarily, step 540 comprises:

Step A: determining an initial value of the docking pose between the first target protein and the second target protein; For example, the initial value can be determined by the spectral initialization method, for specific details, refer to the above description which will not be repeated here.

Step B, based on the initial value of the docking pose between the first target protein and the second target protein, iteratively performing T times of gradient descent based on the difference between distances between reference atoms included in each amino acid residue of the first target protein and reference atoms included in each amino acid residue of the second target protein and the predicted distance map when the first target protein and the second target protein are docked into a protein complex in the initial value of the docking pose, to obtain an approximate solution of the optimal docking pose.

For the specific iteration process, refer to the description of formula (5).

By unfolding gradient descent, examples of the present disclosure can control computational costs during the inference process. When using the spectral initialization method to determine the initial value of the docking pose between the first target protein and the second target protein, it can avoid falling into local optima and find the optimal solution faster, thereby further reducing computational costs during the inference process.

Step 550, taking the 3D structure of the protein complex obtained by docking the first target protein and the second target protein in the optimal docking pose as the predicted 3D structure of the protein complex composed of the first target protein and the second target protein.

As described above, when proteins are regarded as rigid bodies, the protein complex structure prediction problem can be transformed into a rigid body docking problem. After the optimal docking poses $R^*$ and $t^*$ are determined in step 540, in the protein complex formed by docking the first target protein and the second target protein in the optimal docking pose, the coordinates of the i-th reference atom of the first target protein are $X_i$, and the coordinates of the j-th reference atom of the second target protein are $Y_j * R^* + t^*$. Thus, the coordinates of the reference atoms included in each amino acid residue of the first target protein and the coordinates of the reference atoms included in each amino acid residue of the second target protein in the protein complex are determined, i.e., the 3D structural information of the protein complex is determined.

It can be understood that examples of the present disclosure only illustrate the protein complex prediction method by taking a protein complex containing two protein monomers as an example. In fact, the method of examples of the present disclosure can also predict protein complexes formed by three or more protein monomers. Taking the prediction of a complex of four protein monomers as an example, one can first predict the complex formed by two of the protein monomers, then predict the complex formed by this complex and the third protein monomer, and then predict the complex formed by the three protein monomers and the fourth protein monomer; alternatively, one can first predict the complex formed by the first and second protein monomers, and the complex formed by the third and fourth protein monomers, and then predict the complex formed by the two complexes.

In a specific embodiment of the present disclosure, in the training phase, the protein complex structure prediction model is trained using the method 400A for training a protein complex structure prediction model. In the inference phase, prediction is performed using the protein complex structure prediction method 400B, which includes steps 410B-450B, as shown in FIG. 4B.

Step 410B: obtaining input data, wherein the input data comprises a first target amino acid sequence of a first target protein, a second target amino acid sequence of a second target protein, monomeric 3D structural information of the first target protein, and monomeric 3D structural information of the second target protein;

Step 420B: determining input features corresponding to the input data based on the first target amino acid sequence, the second target amino acid sequence, the monomeric 3D structural information of the first target protein, and the monomeric 3D structural information of the second target protein;

Step 430B: inputting the input features corresponding to the input data into the protein complex structure prediction model trained according to the method 400A to obtain a prediction result; wherein the prediction result comprises a predicted distance map; the predicted distance map is a 2D matrix, wherein an element at the i-th row and the j-th column of the predicted distance map represents a predicted distance between the i-th reference atom of the first target protein and the j-th reference atom of the second target protein when the first target protein and the second target protein form a protein complex, as predicted by the protein complex structure prediction model;

Step 440B: determining an optimal docking pose of the first target protein and the second target protein, wherein the docking pose of the first target protein and the second target protein when docked into a protein complex is the optimal docking pose, the difference between distances between reference atoms included in each amino acid residue of the first target protein and reference atoms included in each amino acid residue of the second target protein are closest to the predicted distance map;

Step 450B: taking the 3D structure of the protein complex obtained by docking the first target protein and the second target protein in the optimal docking pose as the predicted 3D structure of the protein complex composed of the first target protein and the second target protein.

For descriptions of steps 410B-450B, refer to the descriptions of steps 510-550, which will not be repeated here.

In a specific embodiment of the present disclosure, as shown in FIG. 6, another method 600 for protein complex structure prediction includes steps 610 to 630.

Step 610: inputting amino acid sequences of a plurality of protein chains in a protein complex into a trained position prediction model to obtain predicted atomic positions of first representative atoms of each protein chain;

Step 620: determining an optimal translation-rotation relationship between the plurality of protein chains based on the predicted atomic positions corresponding to the plurality of protein chains and a predicted distance map, such that a distance difference between a calculated distance map of the plurality of protein chains under the optimal translation-rotation relationship and the predicted distance map is minimized; wherein the predicted distance map is determined based on the amino acid sequences of the plurality of protein chains, and is used to characterize approximate distances between second representative atoms of the plurality of protein chains; the calculated distance map is determined based on the predicted atomic positions corresponding to the plurality of protein chains and a translation-rotation relationship between the plurality of protein chains, and is used to characterize distances between first representative atoms of the plurality of protein chains under the translation-rotation relationship; and the second representative atoms and the first representative atoms are the same or different;

Step 630: obtaining a predicted structure of the protein complex based on the optimal translation-rotation relationship.

In step 610, the position prediction model is used to predict the atomic positions of the first representative atoms in each protein chain within the protein complex to obtain the predicted atomic positions of the first representative atoms. For example, if the protein complex includes 3 protein chains, the 3 protein chains can be input separately or simultaneously into the position prediction model to obtain the predicted atomic positions of the first representative atoms corresponding to the 3 protein chains separately or simultaneously. The position prediction model can be pre-trained, and can be any existing protein monomer structure prediction model, such as alphafold2. The prediction result of the position prediction model can include the positions of all atoms in the protein chain. For convenience of subsequent calculations, the positions of the first representative atoms are selected as representative points.

In step 620, it can be understood that when the predicted distance map includes approximate distances of second representative atoms of N protein chains, the approximate distances in the predicted distance map are determined based on the amino acid sequences of these N protein chains.

The predicted distance map contains approximate distances between second representative atoms in at least two different protein chains. For example, if a protein complex includes protein chain A, protein chain B, and protein chain C, the predicted distance map can include approximate distances between second representative atoms in protein chain A and protein chain B, and approximate distances between second representative atoms in protein chain B and protein chain C, or it can include approximate distances between second representative atoms in protein chain A, protein chain B, and protein chain C.

For example, if a protein complex includes protein chain A, protein chain B, and protein chain C, where protein chain A includes a total of three second representative atoms A1, A2, and A3, protein chain B includes a total of three second representative atoms B1, B2, and B3, and protein chain C includes a total of two second representative atoms C1 and C2, then the predicted distance map can be as shown in Table 1 (distances are for illustration only).

Table 1

| | Protein A (including 3 Cb atoms: A1, A2, A3) | | | Protein B (including 3 Cb atoms: B1-B3) | | | Protein C (including 2 Cb atoms: C1, C2) |
|---|---|---|---|---|---|---|---|
| Protein A (including 3 Cb atoms: A1, A2, A3) | Numbers for distances can also be here | | | This part is the same as the distance between A-B | | | This part is the same as the distance between A-C |
| Protein B (including 3 Cb atoms: B1-B3) | 1 | 3 | 52 | Numbers for distances can also be here | | | This part is the same as the distance between C-B |
| | 45 | 35 | 64 | | | | |
| | 24 | 24 | 25 | | | | |
| Protein C (including 2 Cb atoms: C1, C2) | 26 | 33 | 22 | 4 | 26 | 59 | Numbers for distances can also be here |
| | 15 | 19 | 36 | 18 | 36 | 62 | |

When the predicted distance map contains approximate distances of second representative atoms in more than 3 different protein chains, in addition to the 2D matrix form in Table 1, the predicted distance map can also be a multi-dimensional tensor including approximate distances of second representative atoms between pairs of protein chains. For example, if the predicted distance map contains approximate distances of second representative atoms in 3 different protein chains A, B, and C, one layer can represent the approximate distances of second representative atoms in protein chains A and B, and another layer can represent the approximate distances of second representative atoms in protein chains B and C. Optionally, the predicted distance map can also have a layer representing the approximate distances of second representative atoms in protein chains A and C.

The translation-rotation relationship represents the relative translation/rotation amount between multiple protein chains. The calculated distance map is determined based on the predicted atomic positions corresponding to the multiple protein chains and the translation-rotation relationship between the multiple protein chains, and is used to characterize the distances between the first representative atoms of the multiple protein chains under that translation-rotation relationship. Assuming that the protein chains are rigid bodies, given the predicted atomic positions of the first representative atoms of the multiple protein chains is determined, for each translation-rotation relationship, a calculated distance map corresponding to that translation-rotation relationship can be determined, which is used to characterize the distances between the first representative atoms of the multiple protein chains under that translation-rotation relationship.

The first representative atom and the second representative atom can be $\alpha$-carbon atoms, $\beta$-carbon atoms (hereinafter referred to as Cb), or hydrogen atoms or oxygen atoms. The first representative atom and the second representative atom can be atoms that only one existed in an amino acid.

The first representative atom and the second representative atom can be the same or different. When the first representative atom and the second representative atom are different, the distances in the calculated distance map are characterized by the first representative atoms, and the predicted distance map is characterized by the second representative atoms. Since the distance characterization standards of the calculated distance map and the predicted distance map are different, the difference between them cannot be calculated. In this case, both need to be uniformly converted to be characterized by the first representative atoms or by the second representative atoms. Assuming that the protein chains are rigid bodies, the relative positional relationship between the first representative atom and the second representative atom is determined. Therefore, the distance characterized by one type of representative atom can be converted to be characterized by another type of representative atom.

Exemplarily, the calculated distance map can be converted into a converted calculated distance map, wherein the converted calculated distance map is characterized by distances between second representative atoms; the distance difference between the converted calculated distance map and the predicted distance map is minimized of the plurality of protein chains under the optimal translation-rotation relationship is minimized; alternatively, the predicted distance map can be converted into a converted predicted distance map, wherein the converted predicted distance map is characterized by distances between first representative atoms; the distance difference between the calculated distance map of the plurality of protein chains under the optimal translation-rotation relationship and the converted predicted distance map is minimized.

It can be understood that, for each translation-rotation relationship, a calculated distance map corresponding to that translation-rotation relationship can be determined, and thus a distance difference between the calculated distance map and the predicted distance map can be determined. The translation-rotation relationship that minimizes the distance difference is the optimal translation-rotation relationship. Prior art methods can be used as methods for solving the optimal translation-rotation relationship, such as gradient descent, etc.

In step 630, specifically, the optimal translation-rotation relationship is used to indicate the relative translation/-rotation amount between multiple protein chains. Based on the translation amount and rotation amount, the positions of atoms in the protein complex are transformed to obtain the atomic positions under the optimal translation-rotation relationship, thereby obtaining the predicted structure of the protein complex.

Exemplarily, step 630 comprises: performing coordinate transformation on the predicted atomic positions corresponding to the protein chains based on the optimal translation-rotation relationship to obtain the predicted structure of the protein complex.

Specifically, the optimal translation-rotation relationship includes the translation amount and rotation amount that atoms in the protein chains need to move. The predicted atomic positions corresponding to the protein chains are transformed according to the translation amount and rotation amount to obtain target atomic positions. By combining and constructing of each atom according to its target atomic position, the predicted structure of the protein complex can be obtained.

Exemplarily, the predicted distance map in step 620 can be determined based on a neural network. Exemplarily, before determining the optimal translation-rotation relationship between the plurality of protein chains based on the predicted atomic positions corresponding to the plurality of protein chains and the predicted distance map, the method 600 further includes steps 640 and 650.

640: inputting amino acid sequences of the plurality of protein chains into a trained target map prediction network to obtain a quasi-predicted distance map, wherein the quasi-predicted distance map comprises at least one M*N*P tensor, where P is the number of distance categories, and an element value of the element at the mn-th position (i.e., m-th row and n-th column or n-th row and m-th column) of the p-th layer of the matrix represents a probability that a predicted distance between the m-th first representative atom of the first protein chain and the n-th first representative atom of the second protein chain falls into the p-th distance category, and different distance categories correspond to different distance ranges; wherein p is an integer selected from 1 to P; m is an integer selected from 1 to M, and n is an integer selected from 1 to N;

Specifically, the target map prediction network is used to predict the quasi-predicted distance map based on features determined from the amino acid sequences of the protein chains (and the predicted atomic positions of the protein chains).

For example, common distances can be divided into 64 categories, with different distance categories corresponding to different distance ranges, and distances greater than common distances are uniformly classified into the 64th category. The quasi-predicted distance map can be an M*N*64 tensor. For each element in each M*N, it is a 64-classification problem. For example, if the model predicts that the categories corresponding to distance between the first representative atom A1 of protein chain A and the first representative atom B1 of protein chain B has a high probability of belonging to the 53rd category, then among the 64 components corresponding to A1-B1, the element value of the 53rd component is closest to 1 (e.g., 0.9), and the element values of other components are smaller.

650: obtaining the predicted distance map based on the quasi-predicted distance map; wherein the predicted distance map contains approximate distances between the m-th first representative atom of the first protein chain and the n-th first representative atom of the second protein chain, and the approximate distance is represented by the category corresponding to the maximum probability corresponding to the m-th first representative atom of the first protein chain and the n-th first representative atom of the second protein chain.

Specifically, obtaining the predicted distance map based on the quasi-predicted distance map includes: Directly using the quasi-predicted distance map as the predicted distance map; alternatively, compressing the dimension of each layer matrix in the quasi-predicted distance map (e.g., using the distance category corresponding to the largest value among the 64 components corresponding to A1-B1 in the quasi-predicted distance map as the element value corresponding to A1-B1 in the predicted distance map) to obtain a 2D matrix used to indicate distances between first representative atoms in different protein chains, and using this 2D matrix as the predicted distance map.

Compared to complex networks that can predict precise distances between protein chains at once, the target map prediction network in examples of the present disclosure only predicts the category corresponding to the distance, which can greatly reduce model size and training complexity.

Exemplarily, the target map prediction network includes an evolutionary network module (evoformer module) and a map generation module; wherein, before inputting the amino acid sequences of the plurality of protein chains into the trained target map prediction network to obtain the quasi-predicted distance map, the method includes steps P2T1~P2T3 for training the target map prediction network:

P2T1: inputting amino acid sequences of a plurality of training protein chains included in a protein complex with known structure into the evolutionary network module to obtain pairing features.

It can be understood that, in a protein complex with known structure, the amino acid sequences of the training protein chains are known, and the distances between second representative atoms between different protein chains are known (e.g., determined by experimental methods or other reliable computational methods), and the distance map (actual atomic approximate distances) is also known. For example, actual atomic distances can be categorized to obtain actual atomic approximate distances.

Specifically, the pairing features are pair embeddings (a matrix whose dimensions may not be the same as the predicted distance map); wherein, the evolutionary network module may also have protein homologous sequence features, which are not specifically limited here.

P2T2: inputting the pairing features into the map generation module to obtain predicted atomic approximate distances.

Specifically, the shape of the predicted atomic approximate distances is the same as the quasi-predicted distance map or the predicted distance map.

P2T3: adjusting parameters of the evolutionary network module and/or the map generation module based on a difference value between the predicted atomic approximate distances (i.e., predicted categories) and the actual atomic approximate distances (i.e., categories corresponding to actual distances) to obtain the target map prediction network.

It can be understood that the actual atomic approximate distances and the predicted atomic approximate distances have the same shape.

Specifically, calculate the difference value between the predicted atomic approximate distance and the actual atomic approximate distance for every two first representative atoms, and adjust the parameters of the evolutionary network module and/or the map generation module based on this difference value to obtain the target map prediction network.

Exemplarily, when the second representative atom and the first representative atom are different, determining the optimal translation-rotation relationship between the plurality of protein chains based on the predicted atomic positions corresponding to the plurality of protein chains and the predicted distance map, comprising: Converting the calculated distance map into a converted calculated distance map, wherein the converted calculated distance map is characterized by distances between second representative atoms; the distance difference between the converted calculated distance map of the plurality of protein chains under the optimal translation-rotation relationship and the predicted distance map is minimized. Alternatively, converting the predicted distance map into a converted predicted distance map, wherein the converted predicted distance map is characterized by distances between first representative atoms; the distance difference between the calculated distance map of the plurality of protein chains under the optimal translation-rotation relationship and the converted predicted distance map is minimized.

Specifically, since the first representative atom and the second representative atom can be interconverted based on their positions, if characterization by the second representative atom is required, the calculated distance map is converted into a converted calculated distance map, wherein the converted calculated distance map is characterized by distances between second representative atoms; if characterization by the first representative atom is required, the predicted distance map is converted into a converted predicted distance map, wherein the converted predicted distance map is characterized by distances between first representative atoms; regardless of whether characterization is performed by the first representative atom or the second representative atom, it is necessary to minimize the distance difference between the calculated distance map of the plurality of protein chains under the optimal translation-rotation relationship and the converted predicted distance map.

Exemplarily, determining the optimal translation-rotation relationship between the plurality of protein chains based on the predicted atomic positions corresponding to the plurality of protein chains and the predicted distance map, comprising:

Obtaining the optimal translation-rotation relationship according to the following formula:

$$\min_{T} \|\, \|x_A - T \circ x_B\| - C\|, \ T = (R, t)$$

;

wherein, $x_A$ is the predicted atomic position of one protein chain, $x_B$ is the predicted atomic position of another protein chain, C is the predicted distance map, T is the translation-rotation relationship, R is the rotation matrix, and t is the translation matrix.

Specifically, gradient descent method can be used to solve for the minimum value of the difference. $\|x_A - T \circ x_B\|$ is the calculated distance map. The position conversion of the first representative atom can be performed at the $X_A$, $X_B$ and C levels, or after obtaining the calculated distance map.

Exemplarily, when the protein complex includes W protein chains, the optimal translation-rotation relationship includes W-1 translation-rotation relationships, where W is greater than or equal to 3.

It can be understood that when a protein complex includes W protein chains, there is a translation-rotation relationship corresponding to every two protein chains, but no cycles are formed, so there are a total of W-1 translation-rotation relationships. In other words, the optimal translation-rotation relationship can be a set composed of W-1 translation-rotation relationships.

The W-1 translation-rotation relationships minimize the weighted sum of the distance differences between the calculated distance maps of pairs of protein chains and the predicted distance maps.

For example, for a protein complex composed of 3 protein chains A, B, and C, it is necessary to find the optimal translation-rotation relationship T1 between A and B, and the optimal translation-rotation relationship T2 between B and C. T1 and T2 constitute the optimal translation-rotation relationship T. When the weighted sum of the distance difference d1 between the calculated distance map (here referring to the calculated distance map corresponding to A and B, calculated using the predicted atomic positions of A and B and the translation-rotation relationship T11) and the predicted distance map (here referring to the predicted distance map corresponding to A and B, which can be obtained by inputting the amino acid sequences of A and B into the model) under a certain translation-rotation relationship T11 between A and B, and the distance difference d2 between the calculated distance map (here referring to the calculated distance map corresponding to B and C, calculated using the predicted atomic positions of B and C and the translation-rotation relationship T21) and the predicted distance map (here referring to the predicted distance map corresponding to B and C, which can be obtained by inputting the amino acid sequences of B and C into the model) under a certain translation-rotation relationship T21 between B and C, is minimized, T11 is considered to be T1, and T21 is considered to be T2. In other words, T1 and T2 minimize the weighted sum of d1 and d2.

Alternatively, each of the W-1 translation-rotation relationships minimizes the distance difference between the calculated distance map and the predicted distance map for the two protein chains whose relative relationship it represents.

In this case, T1 and T2 are decoupled, i.e., T1 minimizes d1, and T2 minimizes d2.

In this embodiment, when the protein chains include A, B, C, and D (4 chains), the translation-rotation relationship T1 between A and B can be determined first, then the translation-rotation relationship T2 between B and C can be determined, and then the translation-rotation relationship T3 between C and D can be determined. In this case, the changes brought to B after complexing of A and B are not considered.

Exemplarily, when the protein complex includes a third protein chain, a fourth protein chain, and a fifth protein chain, the optimal translation-rotation relationship includes a first optimal translation-rotation relationship between the third protein chain and the fourth protein chain, and a second optimal translation-rotation relationship between the complex and the fifth protein chain; the complex is a complex composed of the third protein chain and the fourth protein chain under the first optimal translation-rotation relationship.

In this case, step 610 comprises: inputting the amino acid sequence of the third protein chain, the amino acid sequence of the fourth protein chain, and the amino acid sequence of the fifth protein chain into a trained position prediction model to obtain predicted atomic positions of first representative atoms in the third protein chain, predicted atomic positions of first representative atoms in the fourth protein chain, and predicted atomic positions of first representative atoms in the fifth protein chain.

Specifically, when the protein complex includes three protein chains, for example, protein chain A, protein chain B, and protein chain C, the amino acid sequences corresponding to protein chain A, protein chain B, and protein chain C are input into a trained position prediction model to obtain the predicted atomic positions of the first representative atoms of protein chain A, the predicted atomic positions of the first representative atoms of protein chain B, and the predicted atomic positions of the first representative atoms of protein chain C.

Step 620 comprises: determining the first optimal translation-rotation relationship between the third protein chain and the fourth protein chain based on the predicted atomic positions of the third protein chain, the predicted atomic positions of the fourth protein chain, and the approximate distances between the second representative atoms of the third protein chain and the fourth protein chain included in the predicted distance map.

Continuing the previous example, based on the predicted atomic positions of protein chain A, the predicted atomic positions of protein chain B, and the predicted distance map, the first optimal translation-rotation relationship T1 between the third protein chain and the fourth protein chain is determined according to the method of step 620. The predicted distance map here only needs to include the approximate distances between the second representative atoms corresponding to protein chain A and protein chain B, and does not need to include the approximate distances between the second representative atoms of AC or between the second representative atoms of BC. Protein chain A and protein chain B form complex AB under T1.

Exemplarily, the protein complex structure prediction method further includes steps 660-680.

Step 660: obtaining predicted atomic positions of first representative atoms in the complex.

For example, given the predicted atomic positions of protein chain A, the predicted atomic positions of protein chain B, and T1 are known, the predicted atomic positions of the first representative atoms in the complex can be calculated.

Step 670: obtaining a complex predicted distance map corresponding to the complex and the fifth protein chain; the complex predicted distance map is determined based on the amino acid sequence of the complex and the amino acid sequence of the fifth protein chain, and the complex predicted distance map is used to characterize approximate distances between second representative atoms of the complex and second representative atoms of the fifth protein chain.

For example, input the amino acid sequence of the complex and the amino acid sequence of protein chain C into the target map prediction network to obtain a complex predicted distance map, wherein the complex predicted distance map is used to characterize approximate distances between second representative atoms of the complex and second representative atoms of protein chain C.

Step 680: determining a second optimal translation-rotation relationship between the complex and the fifth protein chain based on the predicted atomic positions corresponding to the complex, the predicted atomic positions corresponding to the fifth protein chain, and the complex predicted distance map.

In step 680, based on the predicted atomic positions of complex AB, the predicted atomic positions of protein chain C, and the complex predicted distance map, the second optimal translation-rotation relationship is determined according to the method of step 620.

Thereafter, based on the first optimal translation-rotation relationship and the second optimal translation-rotation relationship, the predicted structure of the protein complex is determined according to the method of step 630.

Thus, the calculation of translation-rotation relationships between more than three protein chains can be decomposed into multiple calculations of translation-rotation relationships between pairs of chains. For example, when the protein chains include A, B, C, and D (4 chains), the translation-rotation relationship T1 between A and B can be determined first, then the translation-rotation relationship T2 between AB and C can be determined, and then the translation-rotation relationship T3 between ABC and D can be determined. This approach considers the changes in protein chains after complex formation.

According to one aspect of the present disclosure, an electronic device is provided, comprising: at least one processor; and at least one memory communicatively coupled to the at least one processor, wherein the at least one memory stores instructions which, when executed by the at least one processor, cause the at least one processor to perform the methods described above.

According to another aspect of the present disclosure, a non-transitory computer-readable storage medium storing instructions is provided, wherein the instructions, when executed by at least one processor of a computer, cause the computer to perform the methods described above.

According to another aspect of the present disclosure, a computer program product is provided, comprising a computer program, wherein the computer program, when executed by a processor, implements the methods described above.

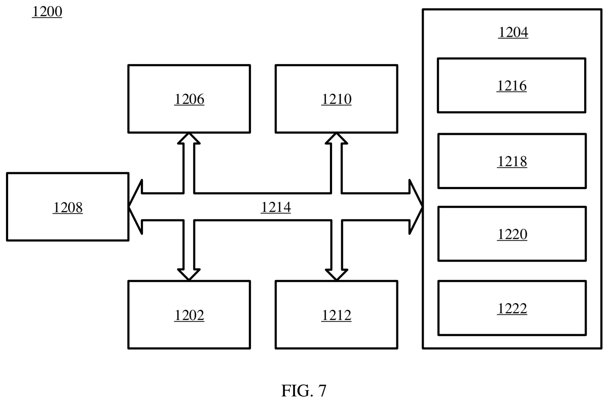

FIG. 7 is a block diagram of an exemplary electronic device capable of implementing examples of the present disclosure.

The electronic device 1200 can be various different types of devices. Examples of the electronic device 1200 include, but are not limited to: desktop computers, server computers, laptop or netbook computers, mobile devices (e.g., tablet computers, cellular or other wireless phones (e.g., smartphones), notebook computers, mobile stations), wearable devices (e.g., glasses, watches), entertainment devices (e.g., entertainment appliances, set-top boxes communicatively coupled to display devices, game consoles), televisions or other display devices, automotive computers, and so on.

The electronic device 1200 may include at least one processor 1202, memory 1204, communication interface(s) 1206, display device 1208, other input/output (I/O) devices 1210, and one or more mass storage devices 1212, capable of communicating with each other, such as via a system bus 1214 or other suitable connections.

The processor 1202 can be a single processing unit or multiple processing units, all of which can include single or multiple computing units or multiple cores. The processor 1202 can be implemented as one or more microprocessors, microcomputers, microcontrollers, digital signal processors, central processing units, state machines, logic circuits, and/or any device that manipulates signals based on operational instructions. Among other capabilities, the processor 1202 can be configured to obtain and execute computer-readable instructions, such as program code of an operating system 1216, program code of an application program 1218, program code of other programs 1220, etc, stored in the memory 1204, mass storage device(s) 1212, or other computer-readable media.

The memory 1204 and the mass storage device(s) 1212 are examples of computer-readable storage media for storing instructions that are executed by the processor 1202 to implement the various functions described above. For example, the memory 1204 may generally include both volatile memory and non-volatile memory (e.g., RAM, ROM, etc.). In addition, the mass storage device(s) 1212 may generally include hard disk drives, solid-state drives, removable media, including external and removable drives, memory cards, flash memory, floppy disks, optical discs (e.g., CD, DVD), storage arrays, network-attached storage, storage area networks, and so on. The memory 1204 and the mass storage device(s) 1212 can both be collectively referred to herein as memory or computer-readable storage media, and can be non-transitory media capable of storing computer-readable, processor-executable program instructions as computer program code, which can be executed by the processor 1202 as a specific machine configured to implement the operations and functions described in the examples herein.

Multiple programs may be stored on the mass storage device(s) 1212. These programs include the operating system 1216, one or more application programs 1218, other programs 1220, and program data 1222, and they can be loaded into the memory 1204 for execution. Examples of such application programs or program modules may include, for example, computer program logic (e.g., computer program code or instructions) for implementing the following components/functions: method 100 (including any suitable steps of method 100), method 400A (including any suitable steps of method 400A), method 400B (including any suitable steps of method 400B), method 500 (including any suitable steps of method 500), method 600 (including any suitable steps of method 600), and/or additional examples described herein.

Although illustrated in FIG. 7 as being stored in the memory 1204 of the electronic device 1200, modules 1216, 1218, 1220, and 1222 or portions thereof may be implemented using any form of computer-readable medium accessible by the electronic device 1200. As used herein, "computer-readable medium" includes at least two types of computer-readable media, namely computer-readable storage media and communication media.

Computer-readable storage media include volatile and non-volatile, removable and non-removable media implemented by any method or technology for storing information, such as computer-readable instructions, data structures, program modules, or other data. Computer-readable storage media include, but are not limited to, RAM, ROM, EEPROM, flash memory or other memory technology, CD-ROM, Digital Versatile Disc (DVD), or other optical storage devices, magnetic cassettes, magnetic tape, disk storage devices or other magnetic storage devices, or any other non-transitory medium that can be used to store information for access by an electronic device. In contrast, communication media may implement computer-readable instructions, data structures, program modules, or other data in a modulated data signal, such as a carrier wave or other transmission mechanism. Computer-readable storage media as defined herein do not include communication media.

One or more communication interfaces 1206 are used to exchange data with other devices, such as via a network, direct connection, etc. Such communication interfaces can be one or more of the following: any type of network interface (e.g., Network Interface Card (NIC)), wired or wireless (such as IEEE 802.11 Wireless LAN (WLAN)) wireless interface, Worldwide Interoperability for Microwave Access (Wi-MAX) interface, Ethernet interface, Universal Serial Bus (USB) interface, cellular network interface, BluetoothTM interface, Near Field Communication (NFC) interface, etc. The communication interfaces 1206 can facilitate communication within various network and protocol types, including wired networks (e.g., LAN, cable, etc.) and wireless networks (e.g., WLAN, cellular, satellite, etc.), the Internet, etc. The communication interfaces 1206 can also provide communication with external storage devices (not shown) such as storage arrays, network-attached storage, storage area networks, etc.

In some examples, a display device 1208, such as a monitor and so on, may be included for displaying information and images to a user. Other I/O devices 1210 can be devices that receive various inputs from a user and provide various outputs to the user, and may include touch input devices, gesture input devices, cameras, keyboards, remote controls, mice, printers, audio input/output devices, and so on.

The techniques described herein can be supported by these various configurations of the electronic device 1200 and are not limited to the specific examples of the techniques described herein. For example, the functionality can also be implemented wholly or partially on the "cloud" using distributed systems. The cloud includes and/or represents a platform for resources. The platform abstracts the underlying functionality of the cloud's hardware (e.g., servers) and software resources. Resources can include applications and/or data that can be used when performing computing processing on servers remote from the electronic device 1200. Resources can also include services provided over the Internet and/or through subscriber networks such as cellular or Wi-Fi networks. The platform can abstract resources and functions to connect the electronic device 1200 with other electronic devices. Therefore, the implementation of the functionality described herein can be distributed throughout the cloud. For example, functionality can be implemented partly on the electronic device 1200 and partly through a platform that abstracts the functionality of the cloud.

Although the present disclosure has been illustrated and described in detail in the accompanying drawings and the foregoing description, such illustration and description are to be considered illustrative and exemplary, and not restrictive; the present disclosure is not limited to the disclosed examples. By studying the drawings, the disclosure and the appended claims, those skilled in the art will be able to understand and implement variations to the disclosed examples when practicing the claimed subject matter. In the claims, the word "comprising" does not exclude other elements or steps that are not listed, the indefinite article "a" or "an" does not exclude a plurality, and the term "plurality" means two or more. The mere fact that certain measures are recited in mutually different dependent claims does not indicate that a combination of these measures cannot be used to advantage.

## Claims

1. A method for training a protein complex structure prediction model, wherein the method comprises:

   obtaining a ground truth distance map corresponding to current training data; wherein the current training data comprises a first amino acid sequence of a first protein, a second amino acid sequence of a second protein, monomeric three-dimensional (3D) structural information of the first protein, monomeric 3D structural information of the second protein, and 3D structural information of a ground truth protein complex composed of the first protein and the second protein; wherein the monomeric 3D structural information of the first protein comprises coordinates of reference atoms included in each amino acid residue of the first protein; the monomeric 3D structural information of the second protein comprises coordinates of reference atoms included in each amino acid residue of the second protein; the 3D structural information of the ground truth protein complex comprises first ground truth coordinates of reference atoms included in each amino acid residue of the first protein and second ground truth coordinates of reference atoms included in each amino acid residue of the second protein in the ground truth protein complex; and the ground truth distance map is determined based on the first ground truth coordinates and the second ground truth coordinates;
   obtaining input features corresponding to the current training data; wherein the input features are determined based on the first amino acid sequence, the second amino acid sequence, the monomeric 3D structural information of the first protein, and the monomeric 3D structural information of the second protein;
   performing model prediction, comprising: inputting the input features into a protein complex structure prediction model having current parameters to obtain a prediction result; wherein the prediction result comprises a predicted distance map; the predicted distance map and the ground truth distance map comprise two-dimensional (2D) matrices, wherein an element at an i-th row and a j-th column of the predicted distance map represents a predicted distance between an i-th reference atom of the first protein and a j-th reference atom of the second protein when the first protein and the second protein form a protein complex, as predicted by the protein complex structure prediction model; and an element at the i-th row and the j-th column of the ground truth distance map represents a ground truth distance between the i-th reference atom of the first protein and the j-th reference atom of the second protein in the ground truth protein complex;
   updating the current parameters based on a loss function to train and obtain the protein complex structure prediction model; wherein the loss function comprises a distance loss function; the distance loss function is used to characterize a mapping relationship between a first difference and the parameters of the protein complex structure prediction model; and the first difference is a difference between the predicted distance map and the ground truth distance map.

2. The method according to claim 1, wherein the loss function further comprises an inner loss function, wherein the inner loss function is used to characterize a mapping relationship between a second difference and an optimal docking pose; the second difference is a difference between distances between reference atoms included in each amino acid residue of the first protein and reference atoms included in each amino acid residue of the second protein when the first protein and the second protein are docked into a protein complex in the optimal docking pose, and the ground truth distance map; and the method further comprises:

determining the optimal docking pose of the first protein and the second protein under the current parameters, wherein, under the current parameters, when the first protein and the second protein are docked into a protein complex in the optimal docking pose, a difference between distances between reference atoms included in each amino acid residue of the first protein and reference atoms included in each amino acid residue of the second protein and the predicted distance map is less than a difference when docked in a non-optimal docking pose.

3. The method according to claim 2, wherein said determining the optimal docking pose of the first protein and the second protein under the current parameters comprises: based on an initial value of the docking pose, iteratively updating the initial value of the docking pose through T times of gradient descent based on a difference between distances between reference atoms included in each amino acid residue of the first protein and reference atoms included in each amino acid residue of the second protein and the predicted distance map when the first protein and the second protein are docked into a protein complex in the initial value of the docking pose, to obtain an approximate solution of the optimal docking pose.

4. The method according to claim 3, wherein the initial value of the docking pose is determined based on a spectral initialization method.

5. The method according to any one of claims 1-4, wherein the input features comprise sequence modality features and structural modality features;

the sequence modality features comprise type features and sequence pairing features, wherein the sequence modality features are determined based on the first amino acid sequence and the second amino acid sequence; the type features are used to characterize amino acid types at each position in the first amino acid sequence and the second amino acid sequence; the sequence pairing features are used to characterize positional relationships between amino acids on the first amino acid sequence and the second amino acid sequence;

the structural modality features comprise structural pairing features and angle features; wherein the structural modality features are determined based on the monomeric 3D structural information of the first protein and the monomeric 3D structural information of the second protein; the structural pairing features are used to characterize distances between amino acids on the first amino acid sequence and the second amino acid sequence; the angle features are used to characterize torsion angles of a main chain backbone and side chains of the first protein and the second protein;

the protein complex structure prediction model comprises a multi-layer perceptron and an evolutionary module; inputting the input features into the protein complex structure prediction model having current parameters to obtain a prediction result, comprising:

processing sequence pairing features and the structural pairing features using the multi-layer perceptron to obtain integrated first features;

processing the type features and the angle features using the multi-layer perceptron to obtain integrated second features;

inputting the integrated first features and the integrated second features into the evolutionary module to obtain evolved first features and evolved second features;

determining the predicted distance map based on the evolved first features.

6. The method according to claim 5, wherein the sequence modality features further comprise multiple sequence features; wherein the multiple sequence features are determined as follows:

performing multiple sequence alignment on the first amino acid sequence and the second amino acid sequence to obtain multiple sequence alignment results, performing a masking operation on the multiple sequence alignment results, and performing feature extraction on results of the masking operation to obtain the multiple sequence features; the multiple sequence features are used to characterize amino acid types and amino acid masking status in the multiple sequence alignment results of the first amino acid sequence and the second amino acid sequence; and the prediction result further comprises reconstructing multiple sequences;

said processing the type features and the angle features using the multi-layer perceptron to obtain integrated second features, comprises:

processing the type features, the multiple sequence features, and the angle features using the multi-layer perceptron to obtain integrated second features;

inputting the input features into the protein complex structure prediction model having current parameters to obtain a prediction result, further comprises:

determining the reconstructed multiple sequences based on the evolved second features;

wherein the loss function further comprises a reconstruction loss function; and the reconstruction loss function is used to characterize a mapping relationship between a difference between the reconstructed multiple sequences and the multiple sequence alignment results and the parameters of the protein complex structure prediction model.

7. A method for predicting protein complex structure, wherein the method comprises:

obtaining input data, wherein the input data comprises a first target amino acid sequence of a first target protein, a second target amino acid sequence of a second target protein, monomeric three-dimensional (3D) structural information of the first target protein, monomeric 3D structural information of the second target protein, wherein the monomeric 3D structural information of the first target protein comprises coordinates of reference atoms included in each amino acid residue of the first target protein; the monomeric 3D structural information of the second target protein comprises coordinates of reference atoms included in each amino acid residue of the second target protein;

determining input features corresponding to the input data based on the first target amino acid sequence, the second target amino acid sequence, the monomeric 3D structural information of the first target protein, and the monomeric 3D structural information of the second target protein;

inputting the input features corresponding to the input data into the protein complex structure prediction model trained by the method according to any one of claims 1-6 to obtain a prediction result; wherein the prediction result comprises a predicted distance map; the predicted distance map comprise 2D matrices, wherein an element at an i-th row and a j-th column of the predicted distance map represents a predicted distance between an i-th reference atom of the first target protein and a j-th reference atom of the second target protein when the first target protein and the second target protein form a protein complex, as predicted by the protein complex structure prediction model;

determining an optimal docking pose of the first target protein and the second target protein, wherein when the first target protein and the second target protein are docked into a protein complex in the optimal docking pose, a difference between distances between reference atoms included in each amino acid residue of the first target protein and reference atoms included in each amino acid residue of the second target protein and a target predicted distance map is less than a difference when docked in a non-optimal docking pose, and the target predicted distance map is determined based on the predicted distance map;

taking a 3D structure of the protein complex obtained by docking the first target protein and the second target protein in the optimal docking pose as a predicted 3D structure of the protein complex composed of the first target protein and the second target protein.

8. The method according to claim 7, wherein said determining an optimal docking pose of the first target protein and the second target protein comprises: based on an initial value of the docking pose, iteratively updating the initial value of the docking pose through T times of gradient descent based on a difference between distances between reference atoms included in each amino acid residue of the first target protein and reference atoms included in each amino acid residue of the second target protein and the predicted distance map when the first target protein and the second target protein are docked into a protein complex in the initial value of the docking pose, to obtain an approximate solution of the optimal docking pose.

9. The method according to claim 8, wherein the initial value of the docking pose is determined based on a spectral initialization method.

10. A computing device comprising:

a memory, a processor and a computer program stored on the memory,

wherein the processor is configured to execute the computer program to implement the steps of the method according to any one of claims 1-8.

11. A non-transitory computer-readable storage medium, having a computer program stored thereon, wherein the computer program, when executed by a processor, implements the steps of the method according to any one of claims 1-8.

12. A computer program product, comprising computer instructions, wherein the computer instructions, when executed by a processor, implement the steps of the method according to any one of claims 1-8.

<u>100</u>

FIG. 1

FIG. 2

FIG. 3

400A

Obtain a ground truth distance map corresponding to current training data — 410A

Obtain input features corresponding to the current training data — 420A

Model prediction — 430A

Determine the optimal docking pose of the first protein and the second protein under the current parameters — 440A

Update the current parameters based on an outer loss function — 450A

Update the current training data — 460A

FIG. 4A

400B

Obtain input data — 410B

Determine input features corresponding to the input data based on the first target amino acid sequence, the second target amino acid sequence, the monomeric 3D structural information of the first target protein, and the monomeric 3D structural information of the second target protein — 420B

Input the input features corresponding to the input data into the protein complex structure prediction model to obtain a prediction result — 430B

Determine an optimal docking pose of the first target protein and the second target protein — 440B

Take the 3D structure of the protein complex obtained by docking the first target protein and the second target protein in the optimal docking pose as the predicted 3D structure of the protein complex composed of the first target protein and the second target protein — 450B

FIG. 4B

500

```
┌─────────────────────────────────────────────────────────┐
│                   Obtain input data                      │────  510
└─────────────────────────────────────────────────────────┘
                            │
                            ▼
┌─────────────────────────────────────────────────────────┐
│ Determine input features corresponding to the input data │
│ based on the first target amino acid sequence, the       │────  520
│ second target amino acid sequence, the monomeric 3D      │
│ structural information of the first target protein, and   │
│ the monomeric 3D structural information of the second     │
│ target protein                                           │
└─────────────────────────────────────────────────────────┘
                            │
                            ▼
┌─────────────────────────────────────────────────────────┐
│ Input the input features corresponding to the input data │
│ into the protein complex structure prediction model to   │────  530
│ obtain a prediction result                               │
└─────────────────────────────────────────────────────────┘
                            │
                            ▼
┌─────────────────────────────────────────────────────────┐
│ Determine an optimal docking pose of the first target    │
│ protein and the second target protein                    │────  540
└─────────────────────────────────────────────────────────┘
                            │
                            ▼
┌─────────────────────────────────────────────────────────┐
│ Take the 3D structure of the protein complex obtained by │
│ docking the first target protein and the second target   │
│ protein in the optimal docking pose as the predicted 3D  │────  550
│ structure of the protein complex composed of the first   │
│ target protein and the second target protein             │
└─────────────────────────────────────────────────────────┘
```

FIG. 5

600

```
┌─────────────────────────────────────────────────────────┐
│ Input amino acid sequences of a plurality of protein     │
│ chains in a protein complex into a trained position      │────  610
│ prediction model to obtain predicted atomic positions of │
│ first representative atoms of each protein chain          │
└─────────────────────────────────────────────────────────┘
                            │
                            ▼
┌─────────────────────────────────────────────────────────┐
│ Determine an optimal translation-rotation relationship   │
│ between the plurality of protein chains based on the     │
│ predicted atomic positions corresponding to the plurality│
│ of protein chains and a predicted distance map, such that │────  620
│ a distance difference between a calculated distance map   │
│ of the plurality of protein chains under the optimal     │
│ translation-rotation relationship and the predicted       │
│ distance map is minimized                                │
└─────────────────────────────────────────────────────────┘
                            │
                            ▼
┌─────────────────────────────────────────────────────────┐
│ Obtain a predicted structure of the protein complex based│
│ on the optimal translation-rotation relationship          │────  630
└─────────────────────────────────────────────────────────┘
```

FIG. 6

FIG. 7

## INTERNATIONAL SEARCH REPORT

| International application No. |
| --- |
| **PCT/CN2024/073357** |

### A. CLASSIFICATION OF SUBJECT MATTER

G16B15/00(2019.01)i

According to International Patent Classification (IPC) or to both national classification and IPC

### B. FIELDS SEARCHED

Minimum documentation searched (classification system followed by classification symbols)

IPC:G16B

Documentation searched other than minimum documentation to the extent that such documents are included in the fields searched

Electronic data base consulted during the international search (name of data base and, where practicable, search terms used)

CNTXT, ENTXTC, VEN, DWPI, CNKI, 必应, BING, 百度学术, BAIDU SCHOLAR, BioRxiv: 蛋白质, 对接, 复合物, 间隔, 间距, 距离, 长度, 位置, 坐标, 原子, 骨架, 基准, 代表, 预测, 氨基酸, 残基, 对接, 扭转角, 进化, 优化, 结构模态, 序列模态, 真实, 实际, Evoformer, protein, complex, backbone, distance, location, coordinator, predict, ture, actual, atom, sequence, structure, dock+

### C. DOCUMENTS CONSIDERED TO BE RELEVANT

| Category* | Citation of document, with indication, where appropriate, of the relevant passages | Relevant to claim No. |
| --- | --- | --- |
| PX | LUO, Yujie et al. "xTrimoDock: Rigid Protein Docking via Cross-Modal Representation Learning and Spectral Algorithm" *BioRxiv preprint doi: https://doi.org/10.1101/2023.02.06.527251*, 06 February 2023 (2023-02-06), sections 2-4 | 1-4, 7-12 |
| X | Anonymous authors. "xTrimoDock:Cross-Modal Tansformer For Multi-Chain Protein Docking" *Under review as a conference paper at ICLR 2023*, 22 September 2022 (2022-09-22), sections 2-4 | 1-4, 7-12 |
| PX | WANG, Ruijia et al. "Injecting Multimodal Information into Rigid Protein Docking via Bi-level Optimization" *37th Conference on Neural Information Processing Systems*, 27 September 2023 (2023-09-27), sections 2-3 | 1-12 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **12 March 2024** | **05 April 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/073357** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 113593633 A (CHINA UNIVERSITY OF PETROLEUM (EAST CHINA)) 02 November 2021 (2021-11-02)<br>      entire document | 1-12 |
| A | CN 115938469 A (SHENZHEN UNIVERSITY) 07 April 2023 (2023-04-07)<br>      entire document | 1-12 |
| A | CN 116052758 A (BAITU SHENGKE (BEIJING) INTELLIGENT TECHNOLOGY CO., LTD.) 02 May 2023 (2023-05-02)<br>      entire document | 1-12 |
| A | US 2021166779 A1 (DEEPMIND TECHNOLOGY LTD.) 03 June 2021 (2021-06-03)<br>      entire document | 1-12 |
| A | US 2021304847 A1 (DEEPMIND TECHNOLOGY LTD.) 30 September 2021 (2021-09-30)<br>      entire document | 1-12 |
| A | US 2023154561 A1 (THE CURATORS OF THE UNIVERSITY OF MISSOURI) 18 May 2023 (2023-05-18)<br>      entire document | 1-12 |

Form PCT/ISA/210 (second sheet) (July 2022)

## INTERNATIONAL SEARCH REPORT
### Information on patent family members

International application No.

**PCT/CN2024/073357**

| Patent document cited in search report | | | Publication date (day/month/year) | Patent family member(s) | | | Publication date (day/month/year) |
|---|---|---|---|---|---|---|---|
| CN | 113593633 | A | 02 November 2021 | CN | 113593633 | B | 25 July 2023 |
| CN | 115938469 | A | 07 April 2023 | None | | | |
| CN | 116052758 | A | 02 May 2023 | None | | | |
| US | 2021166779 | A1 | 03 June 2021 | EP | 4018449 | A1 | 29 June 2022 |
| | | | | WO | 2021110730 | A1 | 10 June 2021 |
| | | | | CN | 114503203 | A | 13 May 2022 |
| US | 2021304847 | A1 | 30 September 2021 | CA | 3110200 | A1 | 26 March 2020 |
| | | | | CA | 3110200 | C | 08 August 2023 |
| | | | | CA | 3110242 | A1 | 26 March 2020 |
| | | | | CA | 3110242 | C | 01 August 2023 |
| | | | | CA | 3110395 | A1 | 26 March 2020 |
| | | | | CA | 3110395 | C | 01 August 2023 |
| | | | | JP | 2022169654 | A | 09 November 2022 |
| | | | | JP | 7389189 | B2 | 29 November 2023 |
| | | | | JP | 2022501696 | A | 06 January 2022 |
| | | | | JP | 7125544 | B2 | 24 August 2022 |
| | | | | JP | 2022501694 | A | 06 January 2022 |
| | | | | JP | 7128346 | B2 | 30 August 2022 |
| | | | | WO | 2020058177 | A1 | 26 March 2020 |
| | | | | US | 2021313008 | A1 | 07 October 2021 |
| | | | | WO | 2020058176 | A1 | 26 March 2020 |
| | | | | US | 2021407625 | A1 | 30 December 2021 |
| | | | | WO | 2020058174 | A1 | 26 March 2020 |
| | | | | EP | 3821433 | A1 | 19 May 2021 |
| | | | | EP | 3821434 | A1 | 19 May 2021 |
| | | | | EP | 3821435 | A1 | 19 May 2021 |
| | | | | JP | 2022501695 | A | 06 January 2022 |
| | | | | JP | 7132430 | B2 | 06 September 2022 |
| | | | | CN | 112585685 | A | 30 March 2021 |
| | | | | IN | 202127003862 | A | 08 October 2021 |
| | | | | IN | 473316 | B | 01 December 2023 |
| US | 2023154561 | A1 | 18 May 2023 | None | | | |

Form PCT/ISA/210 (patent family annex) (July 2022)